# EUROPEAN PATENT SPECIFICATION

(11) **EP 2 392 208 B1**
(45) Date of publication and mention of the grant of the patent: **04.05.2016**
(21) Application number: 10005863.5
(22) Date of filing: 07.06.2010
(51) Int. Cl.: A01K 67/027, C07K 14/415, C12N 15/85, C12N 15/82, C12N 15/62

(54) **Fusion proteins comprising a DNA-binding domain of a Tal effector protein and a non-specific cleavage domain of a restriction nuclease and their use**
Fusionsproteine, die eine DNA-Bindungsdomäne eines TAL-Effektorproteins und eine unspezifische Spaltungsdomäne einer Restriktionsnuklease umfassen, und deren Verwendung
Protéines de fusion comportant un domaine de liaison ADN d'une protéine TAL effectrice et domaine de clivage non spécifique d'une nucléase de restriction et leur utilisation

(43) Date of publication of application: 07.12.2011
(73) Proprietor: Helmholtz Zentrum München Deutsches Forschungszentrum für Gesundheit und Umwelt (GmbH), 85764 Neuherberg (DE)
(72) Inventor: Kühn, Ralf, 13187 Berlin (DE); Wurst, Wolfgang, 80937 München (DE); Meyer, Melanie, 82140 Olching (DE)
(74) Representative: Vossius & Partner Patentanwälte Rechtsanwälte mbB

(56) References cited:
- EP-A1- 2 379 583
- MOEHLE E A ET AL: "Targeted gene addition into a specified location in the human genome using designed zinc finger nucleases" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0611478104, vol. 104, no. 9, 1 February 2007 (2007-02-01), pages 3055-3060, XP002518477 ISSN: 0027-8424
- BOCH JENS ET AL: "Breaking the Code of DNA Binding Specificity of TAL-Type III Effectors" SCIENCE, AMERICAN ASSOCIATION FOR THE ADVANCEMENT OF SCIENCE, WASHINGTON, DC; US LNKD- DOI:10.1126/SCIENCE.1178811, vol. 326, no. 5959, 1 December 2009 (2009-12-01), pages 1509-1512, XP002570745 ISSN: 0036-8075
- TOWNSEND JEFFREY A ET AL: "High-frequency modification of plant genes using engineered zinc-finger nucleases" NATURE, NATURE PUBLISHING GROUP, LONDON, GB LNKD- DOI:10.1038/NATURE07845, vol. 459, no. 7245, 21 May 2009 (2009-05-21), pages 442-445, XP002588972 ISSN: 0028-0836
- MOSCOU MATTHEW J ET AL: "A simple cipher governs DNA recognition by TAL effectors." SCIENCE (NEW YORK, N.Y.) 11 DEC 2009 LNKD- PUBMED:19933106, vol. 326, no. 5959, 11 December 2009 (2009-12-11), page 1501, XP002599998 ISSN: 1095-9203
- GEURTS ARON M ET AL: "Knockout Rats via Embryo Microinjection of Zinc-Finger Nucleases" SCIENCE, AMERICAN ASSOCIATION FOR THE ADVANCEMENT OF SCIENCE, WASHINGTON, DC; US LNKD- DOI:10.1126/SCIENCE.1172447, vol. 325, no. 5939, 24 July 2009 (2009-07-24), page 433, XP002580718 ISSN: 0036-8075 -& GEURTS ARON M ET AL: "Supporting Material for Knockout Rats via Embryo Microinjection of Zinc-Finger Nucleases" SCIENCE, vol. 325, no. 433, 24 July 2009 (2009-07-24), pages 1-13, XP002599999 DOI: 10.1126/science.1172447
- OLSEN PETTER A ET AL: "Analysis of illegitimate genomic integration mediated by zinc-finger nucleases: implications for specificity of targeted gene correction." BMC MOLECULAR BIOLOGY 2010 LNKD- PUBMED:20459736, vol. 11, no. 35, May 2010 (2010-05), pages 1-11, XP002600000 ISSN: 1471-2199
- VOYTAS DANIEL F ET AL: "Plant science. DNA binding made easy", SCIENCE, AMERICAN ASSOCIATION FOR THE ADVANCEMENT OF SCIENCE, US, vol. 326, no. 5959, 11 December 2009 (2009-12-11), pages 1491-1492, XP002632808, ISSN: 1095-9203, DOI: 10.1126/SCIENCE.1183604

### SEQUENCE LISTING

<110> Helmholtz Zentrum Munchen - Deutsches Forschungszentrum fur Gesundheit und Umwelt (GmbH)
<120> Fusion proteins comprising a DNA-binding domain of a Tal effector protein and a non-specific cleavage domain of a restriction nuclease and their use
<130> S1996 EP
<160> 7
<170> PatentIn version 3.4
<210> 1
   <211> 54
   <212> DNA
   <213> Artificial Sequence
<220>
   <223> /note="Description of artificial sequence: tal-finger nuclease target sequence within Rosa26"
<400> 1
   tcgtgatctg caactccagt ctttctagaa gatgggcggg agtcttctgg gcag 54
<210> 2
   <211> 7935
   <212> DNA
   <213> Artificial Sequence
<220>
   <223> /note="Description of artificial sequence: Sequence of Plasmid
   pCAG-venus-TalRosa1-Fok-EL"
<400> 2
   gggtaccggg ccccccctcg aggtcgacgg tatcgataag cttgatatcg aattcgagct     60
   cggtacccgg gggcgcgccg gatctcgaca ttgattattg actagttatt aatagtaatc    120
   aattacgggg tcattagttc atagcccata tatggagttc cgcgttacat aacttacggt    180
   aaatggcccg cctggctgac cgcccaacga cccccgccca ttgacgtcaa taatgacgta    240
   tgttcccata gtaacgccaa tagggacttt ccattgacgt caatgggtgg actatttacg    300
   gtaaactgcc cacttggcag tacatcaagt gtatcatatg ccaagtacgc cccctattga    360
   cgtcaatgac ggtaaatggc ccgcctggca ttatgcccag tacatgacct tatgggactt    420
   tcctacttgg cagtacatct acgtattagt catcgctatt accatgggtc gaggtgagcc    480
   ccacgttctg cttcactctc cccatctccc ccccctcccc acccccaatt ttgtatttat    540
   ttatttttta attattttgt gcagcgatgg gggcgggggg ggggggggcg cgcgccaggc    600
   ggggcggggc ggggcgaggg gcggggcggg gcgaggcgga gaggtgcggc ggcagccaat    660
   cagagcggcg cgctccgaaa gtttcctttt atggcgaggc ggcggcggcg gcggccctat    720
   aaaaagcgaa gcgcgcggcg ggcgggagtc gctgcgttgc cttcgccccg tgccccgctc    780
   cgcgccgcct cgcgccgccc gccccggctc tgactgaccg cgttactccc acaggtgagc    840
   gggcgggacg gcccttctcc tccgggctgt aattagcgct tggtttaatg acggctcgtt    900
   tcttttctgt ggctgcgtga aagccttaaa gggctccggg agggcccttt gtgcgggggg 960
   gagcggctcg gggggtgcgt gcgtgtgtgt gtgcgtgggg agcgccgcgt gcggcccgcg 1020
   ctgcccggcg gctgtgagcg ctgcgggcgc ggcgcggggc tttgtgcgct ccgcgtgtgc 1080
   gcgaggggag cgcggccggg ggcggtgccc cgcggtgcgg gggggctgcg aggggaacaa 1140
   aggctgcgtg cggggtgtgt gcgtgggggg gtgagcaggg ggtgtgggcg cggcggtcgg 1200
   gctgtaaccc ccccctgcac ccccctcccc gagttgctga gcacggcccg gcttcgggtg 1260
   cggggctccg tgcggggcgt ggcgcggggc tcgccgtgcc gggcgggggg tggcggcagg 1320
   tgggggtgcc gggcggggcg gggccgcctc gggccgggga gggctcgggg gaggggcgcg 1380
   gcggccccgg agcgccggcg gctgtcgagg cgcggcgagc cgcagccatt gccttttatg 1440
   gtaatcgtgc gagagggcgc agggacttcc tttgtcccaa atctggcgga gccgaaatct 1500
   gggaggcgcc gccgcacccc ctctagcggg cgcgggcgaa gcggtgcggc gccggcagga 1560
   aggaaatggg cggggagggc cttcgtgcgt cgccgcgccg ccgtcccctt ctccatctcc 1620
   agcctcgggg ctgccgcagg gggacggctg ccttcggggg ggacggggca gggcggggtt 1680
   cggcttctgg cgtgtgaccg gcggctctag agcctctgct aaccatgttc atgccttctt 1740
   ctttttccta cagatcctta attaataata cgactcacta taggggccgc caccatgccc 1800
   aagaagaaga ggaaggtgat ggtgagcaag ggcgaggagc tgttcaccgg ggtggtgccc 1860
   atcctggtcg agctggacgg cgacgtaaac ggccacaagt tcagcgtgtc cggcgagggc 1920
   gagggcgatg ccacctacgg caagctgacc ctgaagctga tctgcaccac cggcaagctg 1980
   cccgtgccct ggcccaccct cgtgaccacc ctgggctacg gcctgcagtg cttcgcccgc 2040
   taccccgacc acatgaagca gcacgacttc ttcaagtccg ccatgcccga aggctacgtc 2100
   caggagcgca ccatcttctt caaggacgac ggcaactaca agacccgcgc cgaggtgaag 2160
   ttcgagggcg acaccctggt gaaccgcatc gagctgaagg gcatcgactt caaggaggac 2220
   ggcaacatcc tggggcacaa gctggagtac aactacaaca gccacaacgt ctatatcacc 2280
   gccgacaagc agaagaacgg catcaaggcc aacttcaaga tccgccacaa catcgaggac 2340
   ggcggcgtgc agctcgccga ccactaccag cagaacaccc ccatcggcga cggccccgtg 2400
   ctgctgcccg acaaccacta cctgagctac cagtccgccc tgagcaaaga ccccaacgag 2460
   aagcgcgatc acatggtcct gctggagttc gtgaccgccg ccgggatcac tctcggcatg 2520
   gacgagctgt acaagggcgg aggcggaggc ggaggcacgc gtctggacac cggccagctg 2580
   ctgaagatcg ccaagagggg cggcgtgacc gccgtggagg ccgtgcacgc ctggaggaac 2640
   gccctgaccg gcgcccctct gaacctgacc ggtcagcagg tggtggccat cgccagccac 2700
   gacggcggca agcaggccct ggagaccgtg cagaggctgc tgcctgtgct gtgccaggcc 2760
   cacggcctga ccggtcagca ggtggtggcc atcgccagcc acgacggcgg caagcaggcc 2820
   ctggagaccg tgcagaggct gctgcctgtg ctgtgccagg cccacggcct gaccggtcag 2880
   caggtggtgg ccatcgccag ccacgacggc ggcaagcagg ccctggagac cgtgcagagg 2940
   ctgctgcctg tgctgtgcca ggcccacggc ctgaccggtc agcaggtggt ggccatcgcc 3000
   agcaacaacg gcggcaagca ggccctggag accgtgcaga ggctgctgcc tgtgctgtgc 3060
   caggcccacg gcctgaccgg tcagcaggtg gtggccatcg ccagccacga cggcggcaag 3120
   caggccctgg agaccgtgca gaggctgctg cctgtgctgt gccaggccca cggcctgacc 3180
   ggtcagcagg tggtggccat cgccagccac gacggcggca agcaggccct ggagaccgtg 3240
   cagaggctgc tgcctgtgct gtgccaggcc cacggcctga ccggtcagca ggtggtggcc 3300
   atcgccagcc acgacggcgg caagcaggcc ctggagaccg tgcagaggct gctgcctgtg 3360
   ctgtgccagg cccacggcct gaccggtgag caggtggtgg ccatcgccag caacatcggc 3420
   ggcaagcagg ccctggagac cgtgcagagg ctgctgcctg tgctgtgcca ggcccacggc 3480
   ctgaccggtc agcaggtggt ggccatcgcc agcaacggcg gcggcaagca ggccctggag 3540
   accgtgcaga ggctgctgcc tgtgctgtgc caggcccacg gcctgaccgg tcagcaggtg 3600
   gtggccatcg ccagccacga cggcggcaag caggccctgg agaccgtgca gaggctgctg 3660
   cctgtgctgt gccaggccca cggcctgacc ggtcagcagg tggtggccat cgccagcaac 3720
   ggcggcggca agcaggccct ggagaccgtg cagaggctgc tgcctgtgct gtgccaggcc 3780
   cacggcctga ccggtcagca ggtggtggcc atcgccagca acggcggcgg caagcaggcc 3840
   ctggagaccg tgcagaggct gctgcctgtg ctgtgccagg cccacggcct gaccggtcag 3900
   caggtggtgg ccatcgccag ccacgacggc ggcaagcagg ccctggagac cgtgcagagg 3960
   ctgctgcctg tgctgtgcca ggcccacggc ctgaccggtc agcaggtggt ggccatcgcc 4020
   agcaacggcg gcggcaggcc tgccctggag agcatcgtgg cccagctgag caggcctgac 4080
   cctgccctgg ccggatccgg cggcggcggc ggcggcggcc aactagtcaa aagtgaactg 4140
   gaggagaaga aatctgaact tcgtcataaa ttgaaatatg tgcctcatga atatattgaa 4200
   ttaattgaaa ttgccagaaa ttccactcag gatagaattc ttgaaatgaa ggtaatggaa 4260
   ttttttatga aagtttatgg atatagaggt aaacatttgg gtggatcaag gaaaccggac 4320
   ggagcaattt atactgtcgg atctcctatt gattacggtg tgatcgtgga tactaaagct 4380
   tatagcggag gttataatct gccaattggc caagcagatg aaatggagcg atatgtcgaa 4440
   gaaaatcaaa cacgaaacaa acatctcaac cctaatgaat ggtggaaagt ctatccatct 4500
   tctgtaacgg aatttaagtt tttatttgtg agtggtcact ttaaaggaaa ctacaaagct 4560
   cagcttacac gattaaatca tatcactaat tgtaatggag ctgttcttag tgtagaagag 4620
   cttttaattg gtggagaaat gattaaagcc ggcacattaa ccttagagga agtgagacgg 4680
   aaatttaata acggcgagat aaactttgct agcggatcca cgcgtaaatg attgcagatc 4740
   cactagttct agagctcgct gatcagcctc gactgtgcct tctagttgcc agccatctgt 4800
   tgtttgcccc tcccccgtgc cttccttgac cctggaaggt gccactccca ctgtcctttc 4860
   ctaataaaat gaggaaattg catcgcattg tctgagtagg tgtcattcta ttctgggggg 4920
   tggggtgggg caggacagca agggggagga ttgggaagac aatagcaggc atgctgggga 4980
   tgcggtgggc tctatggctt ctgaggcgga aagaaccagc tggggctcga gatccactag 5040
   ttctagcctc gaggctagag cggccgccac cgcggtggag ctccaattcg ccctatagtg 5100
   agtcgtatta cgcgcgctca ctggccgtcg ttttacaacg tcgtgactgg gaaaaccctg 5160
   gcgttaccca acttaatcgc cttgcagcac atcccccttt cgccagctgg cgtaatagcg 5220
   aagaggcccg caccgatcgc ccttcccaac agttgcgcag cctgaatggc gaatgggacg 5280
   cgccctgtag cggcgcatta agcgcggcgg gtgtggtggt tacgcgcagc gtgaccgcta 5340
   cacttgccag cgccctagcg cccgctcctt tcgctttctt cccttccttt ctcgccacgt 5400
   tcgccggctt tccccgtcaa gctctaaatc gggggctccc tttagggttc cgatttagtg 5460
   ctttacggca cctcgacccc aaaaaacttg attagggtga tggttcacgt agtgggccat 5520
   cgccctgata gacggttttt cgccctttga cgttggagtc cacgttcttt aatagtggac 5580
   tcttgttcca aactggaaca acactcaacc ctatctcggt ctattctttt gatttataag 5640
   ggattttgcc gatttcggcc tattggttaa aaaatgagct gatttaacaa aaatttaacg 5700
   cgaattttaa caaaatatta acgcttacaa tttaggtggc acttttcggg gaaatgtgcg 5760
   cggaacccct atttgtttat ttttctaaat acattcaaat atgtatccgc tcatgagaca 5820
   ataaccctga taaatgcttc aataatattg aaaaaggaag agtatgagta ttcaacattt 5880
   ccgtgtcgcc cttattccct tttttgcggc attttgcctt cctgtttttg ctcacccaga 5940
   aacgctggtg aaagtaaaag atgctgaaga tcagttgggt gcacgagtgg gttacatcga 6000
   actggatctc aacagcggta agatccttga gagttttcgc cccgaagaac gttttccaat 6060
   gatgagcact tttaaagttc tgctatgtgg cgcggtatta tcccgtattg acgccgggca 6120
   agagcaactc ggtcgccgca tacactattc tcagaatgac ttggttgagt actcaccagt 6180
   cacagaaaag catcttacgg atggcatgac agtaagagaa ttatgcagtg ctgccataac 6240
   catgagtgat aacactgcgg ccaacttact tctgacaacg atcggaggac cgaaggagct 6300
   aaccgctttt ttgcacaaca tgggggatca tgtaactcgc cttgatcgtt gggaaccgga 6360
   gctgaatgaa gccataccaa acgacgagcg tgacaccacg atgcctgtag caatggcaac 6420
   aacgttgcgc aaactattaa ctggcgaact acttactcta gcttcccggc aacaattaat 6480
   agactggatg gaggcggata aagttgcagg accacttctg cgctcggccc ttccggctgg 6540
   ctggtttatt gctgataaat ctggagccgg tgagcgtggg tctcgcggta tcattgcagc 6600
   actggggcca gatggtaagc cctcccgtat cgtagttatc tacacgacgg ggagtcaggc 6660
   aactatggat gaacgaaata gacagatcgc tgagataggt gcctcactga ttaagcattg 6720
   gtaactgtca gaccaagttt actcatatat actttagatt gatttaaaac ttcattttta 6780
   atttaaaagg atctaggtga agatcctttt tgataatctc atgaccaaaa tcccttaacg 6840
   tgagttttcg ttccactgag cgtcagaccc cgtagaaaag atcaaaggat cttcttgaga 6900
   tccttttttt ctgcgcgtaa tctgctgctt gcaaacaaaa aaaccaccgc taccagcggt 6960
   ggtttgtttg ccggatcaag agctaccaac tctttttccg aaggtaactg gcttcagcag 7020
   agcgcagata ccaaatactg tccttctagt gtagccgtag ttaggccacc acttcaagaa   7080
   ctctgtagca ccgcctacat acctcgctct gctaatcctg ttaccagtgg ctgctgccag   7140
   tggcgataag tcgtgtctta ccgggttgga ctcaagacga tagttaccgg ataaggcgca   7200
   gcggtcgggc tgaacggggg gttcgtgcac acagcccagc ttggagcgaa cgacctacac   7260
   cgaactgaga tacctacagc gtgagctatg agaaagcgcc acgcttcccg aagggagaaa   7320
   ggcggacagg tatccggtaa gcggcagggt cggaacagga gagcgcacga gggagcttcc   7380
   agggggaaac gcctggtatc tttatagtcc tgtcgggttt cgccacctct gacttgagcg   7440
   tcgatttttg tgatgctcgt caggggggcg gagcctatgg aaaaacgcca gcaacgcggc   7500
   ctttttacgg ttcctggcct tttgctggcc ttttgctcac atgttctttc ctgcgttatc   7560
   ccctgattct gtggataacc gtattaccgc ctttgagtga gctgataccg ctcgccgcag   7620
   ccgaacgacc gagcgcagcg agtcagtgag cgaggaagcg gaagagcgcc caatacgcaa   7680
   accgcctctc cccgcgcgtt ggccgattca ttaatgcagc tggcacgaca ggtttcccga   7740
   ctggaaagcg ggcagtgagc gcaacgcaat taatgtgagt tagctcactc attaggcacc   7800
   ccaggcttta cactttatgc ttccggctcg tatgttgtgt ggaattgtga gcggataaca   7860
   atttcacaca ggaaacagct atgaccatga ttacgccaag cgcgcaatta accctcacta   7920
   aagggaacaa aagct                                                    7935
<210> 3
   <211> 978
   <212> PRT
   <213> Artificial Sequence
<220>
   <223> /note="Description of artificial sequence: Aminoacid sequence of protein
   venus-TalRosa1-Fok-EL"
<400> 3
   Met Pro Lys Lys Lys Arg Lys Val Met Val Ser Lys Gly Glu Glu Leu
   1               5                   10                  15
   Phe Thr Gly Val Val Pro Ile Leu Val Glu Leu Asp Gly Asp Val Asn
               20                  25                  30
   Gly His Lys Phe Ser Val Ser Gly Glu Gly Glu Gly Asp Ala Thr Tyr
           35                  40                  45
   Gly Lys Leu Thr Leu Lys Leu Ile Cys Thr Thr Gly Lys Leu Pro Val
       50                  55                  60
   Pro Trp Pro Thr Leu Val Thr Thr Leu Gly Tyr Gly Leu Gln Cys Phe
   65                  70                  75                  80
   Ala Arg Tyr Pro Asp His Met Lys Gln His Asp Phe Phe Lys Ser Ala
                   85                  90                  95
   Met Pro Glu Gly Tyr Val Gln Glu Arg Thr Ile Phe Phe Lys Asp Asp
   100 105 110
   Gly Asn Tyr Lys Thr Arg Ala Glu Val Lys Phe Glu Gly Asp Thr Leu
   115 120 125
   Val Asn Arg Ile Glu Leu Lys Gly Ile Asp Phe Lys Glu Asp Gly Asn
   130 135 140
   Ile Leu Gly His Lys Leu Glu Tyr Asn Tyr Asn Ser His Asn Val Tyr
   145 150 155 160
   Ile Thr Ala Asp Lys Gln Lys Asn Gly Ile Lys Ala Asn Phe Lys Ile
   165 170 175
   Arg His Asn Ile Glu Asp Gly Gly Val Gln Leu Ala Asp His Tyr Gln
   180 185 190
   Gln Asn Thr Pro Ile Gly Asp Gly Pro Val Leu Leu Pro Asp Asn His
   195 200 205
   Tyr Leu Ser Tyr Gln Ser Ala Leu Ser Lys Asp Pro Asn Glu Lys Arg
   210 215 220
   Asp His Met Val Leu Leu Glu Phe Val Thr Ala Ala Gly Ile Thr Leu
   225 230 235 240
   Gly Met Asp Glu Leu Tyr Lys Gly Gly Gly Gly Gly Gly Gly Thr Arg
   245 250 255
   Leu Asp Thr Gly Gln Leu Leu Lys Ile Ala Lys Arg Gly Gly Val Thr
   260 265 270
   Ala Val Glu Ala Val His Ala Trp Arg Asn Ala Leu Thr Gly Ala Pro
   275 280 285
   Leu Asn Leu Thr Gly Gln Gln Val Val Ala Ile Ala Ser His Asp Gly
   290 295 300
   Gly Lys Gln Ala Leu Glu Thr Val Gln Arg Leu Leu Pro Val Leu Cys
   305 310 315 320
   Gln Ala His Gly Leu Thr Gly Gln Gln Val Val Ala Ile Ala Ser His
   325 330 335
   Asp Gly Gly Lys Gln Ala Leu Glu Thr Val Gln Arg Leu Leu Pro Val
   340 345 350
   Leu Cys Gln Ala His Gly Leu Thr Gly Gln Gln Val Val Ala Ile Ala
   355 360 365
   Ser His Asp Gly Gly Lys Gln Ala Leu Glu Thr Val Gln Arg Leu Leu
   370 375 380
   Pro Val Leu Cys Gln Ala His Gly Leu Thr Gly Gln Gln Val Val Ala
   385 390 395 400
   Ile Ala Ser Asn Asn Gly Gly Lys Gln Ala Leu Glu Thr Val Gln Arg
   405 410 415
   Leu Leu Pro Val Leu Cys Gln Ala His Gly Leu Thr Gly Gln Gln Val
   420 425 430
   Val Ala Ile Ala Ser His Asp Gly Gly Lys Gln Ala Leu Glu Thr Val
   435 440 445
   Gln Arg Leu Leu Pro Val Leu Cys Gln Ala His Gly Leu Thr Gly Gln
   450 455 460
   Gln Val Val Ala Ile Ala Ser His Asp Gly Gly Lys Gln Ala Leu Glu
   465 470 475 480
   Thr Val Gln Arg Leu Leu Pro Val Leu Cys Gln Ala His Gly Leu Thr
   485 490 495
   Gly Gln Gln Val Val Ala Ile Ala Ser His Asp Gly Gly Lys Gln Ala
   500 505 510
   Leu Glu Thr Val Gln Arg Leu Leu Pro Val Leu Cys Gln Ala His Gly
   515 520 525
   Leu Thr Gly Glu Gln Val Val Ala Ile Ala Ser Asn Ile Gly Gly Lys
   530 535 540
   Gln Ala Leu Glu Thr Val Gln Arg Leu Leu Pro Val Leu Cys Gln Ala
   545 550 555 560
   His Gly Leu Thr Gly Gln Gln Val Val Ala Ile Ala Ser Asn Gly Gly
   565 570 575
   Gly Lys Gln Ala Leu Glu Thr Val Gln Arg Leu Leu Pro Val Leu Cys
   580 585 590
   Gln Ala His Gly Leu Thr Gly Gln Gln Val Val Ala Ile Ala Ser His
   595 600 605
   Asp Gly Gly Lys Gln Ala Leu Glu Thr Val Gln Arg Leu Leu Pro Val
   610 615 620
   Leu Cys Gln Ala His Gly Leu Thr Gly Gln Gln Val Val Ala Ile Ala
   625 630 635 640
   Ser Asn Gly Gly Gly Lys Gln Ala Leu Glu Thr Val Gln Arg Leu Leu
   645 650 655
   Pro Val Leu Cys Gln Ala His Gly Leu Thr Gly Gln Gln Val Val Ala
   660 665 670
   Ile Ala Ser Asn Gly Gly Gly Lys Gln Ala Leu Glu Thr Val Gln Arg
   675 680 685
   Leu Leu Pro Val Leu Cys Gln Ala His Gly Leu Thr Gly Gln Gln Val
   690 695 700
   Val Ala Ile Ala Ser His Asp Gly Gly Lys Gln Ala Leu Glu Thr Val
   705 710 715 720
   Gln Arg Leu Leu Pro Val Leu Cys Gln Ala His Gly Leu Thr Gly Gln
   725 730 735
   Gln Val Val Ala Ile Ala Ser Asn Gly Gly Gly Arg Pro Ala Leu Glu
   740 745 750
   Ser Ile Val Ala Gln Leu Ser Arg Pro Asp Pro Ala Leu Ala Gly Ser
   755 760 765
   Gly Gly Gly Gly Gly Gly Gly Gln Leu Val Lys Ser Glu Leu Glu Glu
   770 775 780
   Lys Lys Ser Glu Leu Arg His Lys Leu Lys Tyr Val Pro His Glu Tyr
   785 790 795 800
   Ile Glu Leu Ile Glu Ile Ala Arg Asn Ser Thr Gln Asp Arg Ile Leu
   805 810 815
   Glu Met Lys Val Met Glu Phe Phe Met Lys Val Tyr Gly Tyr Arg Gly
   820 825 830
   Lys His Leu Gly Gly Ser Arg Lys Pro Asp Gly Ala Ile Tyr Thr Val
   835 840 845
   Gly Ser Pro Ile Asp Tyr Gly Val Ile Val Asp Thr Lys Ala Tyr Ser
   850 855 860
   Gly Gly Tyr Asn Leu Pro Ile Gly Gln Ala Asp Glu Met Glu Arg Tyr
   865 870 875 880
   Val Glu Glu Asn Gln Thr Arg Asn Lys His Leu Asn Pro Asn Glu Trp
   885 890 895
   Trp Lys Val Tyr Pro Ser Ser Val Thr Glu Phe Lys Phe Leu Phe Val
   900 905 910
   Ser Gly His Phe Lys Gly Asn Tyr Lys Ala Gln Leu Thr Arg Leu Asn
   915 920 925
   His Ile Thr Asn Cys Asn Gly Ala Val Leu Ser Val Glu Glu Leu Leu
   930 935 940
   Ile Gly Gly Glu Met Ile Lys Ala Gly Thr Leu Thr Leu Glu Glu Val
   945 950 955 960
   Arg Arg Lys Phe Asn Asn Gly Glu Ile Asn Phe Ala Ser Gly Ser Thr
   965 970 975
   Arg Lys
<210> 4
   <211> 8037
   <212> DNA
   <213> Artificial Sequence
<220>
   <223> /note="Description of artificial sequence: Sequence of Plasmid
   pCAG-venus-TalRosa2-Fok-KK"
<400> 4
   gggtaccggg ccccccctcg aggtcgacgg tatcgataag cttgatatcg aattcgagct      60
   cggtacccgg gggcgcgccg gatctcgaca ttgattattg actagttatt aatagtaatc     120
   aattacgggg tcattagttc atagcccata tatggagttc cgcgttacat aacttacggt     180
   aaatggcccg cctggctgac cgcccaacga cccccgccca ttgacgtcaa taatgacgta     240
   tgttcccata gtaacgccaa tagggacttt ccattgacgt caatgggtgg actatttacg     300
   gtaaactgcc cacttggcag tacatcaagt gtatcatatg ccaagtacgc cccctattga     360
   cgtcaatgac ggtaaatggc ccgcctggca ttatgcccag tacatgacct tatgggactt     420
   tcctacttgg cagtacatct acgtattagt catcgctatt accatgggtc gaggtgagcc     480
   ccacgttctg cttcactctc cccatctccc ccccctcccc acccccaatt ttgtatttat     540
   ttatttttta attattttgt gcagcgatgg gggcgggggg ggggggggcg cgcgccaggc     600
   ggggcggggc ggggcgaggg gcggggcggg gcgaggcgga gaggtgcggc ggcagccaat     660
   cagagcggcg cgctccgaaa gtttcctttt atggcgaggc ggcggcggcg gcggccctat     720
   aaaaagcgaa gcgcgcggcg ggcgggagtc gctgcgttgc cttcgccccg tgccccgctc     780
   cgcgccgcct cgcgccgccc gccccggctc tgactgaccg cgttactccc acaggtgagc     840
   gggcgggacg gcccttctcc tccgggctgt aattagcgct tggtttaatg acggctcgtt     900
   tcttttctgt ggctgcgtga aagccttaaa gggctccggg agggcccttt gtgcgggggg     960
   gagcggctcg gggggtgcgt gcgtgtgtgt gtgcgtgggg agcgccgcgt gcggcccgcg    1020
   ctgcccggcg gctgtgagcg ctgcgggcgc ggcgcggggc tttgtgcgct ccgcgtgtgc    1080
   gcgaggggag cgcggccggg ggcggtgccc cgcggtgcgg gggggctgcg aggggaacaa    1140
   aggctgcgtg cggggtgtgt gcgtgggggg gtgagcaggg ggtgtgggcg cggcggtcgg 1200
   gctgtaaccc ccccctgcac ccccctcccc gagttgctga gcacggcccg gcttcgggtg 1260
   cggggctccg tgcggggcgt ggcgcggggc tcgccgtgcc gggcgggggg tggcggcagg 1320
   tgggggtgcc gggcggggcg gggccgcctc gggccgggga gggctcgggg gaggggcgcg 1380
   gcggccccgg agcgccggcg gctgtcgagg cgcggcgagc cgcagccatt gccttttatg 1440
   gtaatcgtgc gagagggcgc agggacttcc tttgtcccaa atctggcgga gccgaaatct 1500
   gggaggcgcc gccgcacccc ctctagcggg cgcgggcgaa gcggtgcggc gccggcagga 1560
   aggaaatggg cggggagggc cttcgtgcgt cgccgcgccg ccgtcccctt ctccatctcc 1620
   agcctcgggg ctgccgcagg gggacggctg ccttcggggg ggacggggca gggcggggtt 1680
   cggcttctgg cgtgtgaccg gcggctctag agcctctgct aaccatgttc atgccttctt 1740
   ctttttccta cagatcctta attaataata cgactcacta taggggccgc caccatgccc 1800
   aagaagaaga ggaaggtgat ggtgagcaag ggcgaggagc tgttcaccgg ggtggtgccc 1860
   atcctggtcg agctggacgg cgacgtaaac ggccacaagt tcagcgtgtc cggcgagggc 1920
   gagggcgatg ccacctacgg caagctgacc ctgaagctga tctgcaccac cggcaagctg 1980
   cccgtgccct ggcccaccct cgtgaccacc ctgggctacg gcctgcagtg cttcgcccgc 2040
   taccccgacc acatgaagca gcacgacttc ttcaagtccg ccatgcccga aggctacgtc 2100
   caggagcgca ccatcttctt caaggacgac ggcaactaca agacccgcgc cgaggtgaag 2160
   ttcgagggcg acaccctggt gaaccgcatc gagctgaagg gcatcgactt caaggaggac 2220
   ggcaacatcc tggggcacaa gctggagtac aactacaaca gccacaacgt ctatatcacc 2280
   gccgacaagc agaagaacgg catcaaggcc aacttcaaga tccgccacaa catcgaggac 2340
   ggcggcgtgc agctcgccga ccactaccag cagaacaccc ccatcggcga cggccccgtg 2400
   ctgctgcccg acaaccacta cctgagctac cagtccgccc tgagcaaaga ccccaacgag 2460
   aagcgcgatc acatggtcct gctggagttc gtgaccgccg ccgggatcac tctcggcatg 2520
   gacgagctgt acaagggcgg aggcggaggc ggaggcacgc gtctggacac cggccagctg 2580
   ctgaagatcg ccaagagggg cggcgtgacc gccgtggagg ccgtgcacgc ctggaggaac 2640
   gccctgaccg gcgcccctct gaacctgacc ggtcagcagg tggtggccat cgccagccac 2700
   gacggcggca agcaggccct ggagaccgtg cagaggctgc tgcctgtgct gtgccaggcc 2760
   cacggcctga ccggtcagca ggtggtggcc atcgccagca acggcggcgg caagcaggcc 2820
   ctggagaccg tgcagaggct gctgcctgtg ctgtgccagg cccacggcct gaccggtcag 2880
   caggtggtgg ccatcgccag caacaacggc ggcaagcagg ccctggagac cgtgcagagg 2940
   ctgctgcctg tgctgtgcca ggcccacggc ctgaccggtc agcaggtggt ggccatcgcc 3000
   agccacgacg gcggcaagca ggccctggag accgtgcaga ggctgctgcc tgtgctgtgc 3060
   caggcccacg gcctgaccgg tgagcaggtg gtggccatcg ccagcaacat cggcggcaag 3120
   caggccctgg agaccgtgca gaggctgctg cctgtgctgt gccaggccca cggcctgacc 3180
   ggtgagcagg tggtggccat cgccagcaac atcggcggca agcaggccct ggagaccgtg 3240
   cagaggctgc tgcctgtgct gtgccaggcc cacggcctga ccggtcagca ggtggtggcc 3300
   atcgccagcc acgacggcgg caagcaggcc ctggagaccg tgcagaggct gctgcctgtg 3360
   ctgtgccagg cccacggcct gaccggtcag caggtggtgg ccatcgccag caacggcggc 3420
   ggcaagcagg ccctggagac cgtgcagagg ctgctgcctg tgctgtgcca ggcccacggc 3480
   ctgaccggtc agcaggtggt ggccatcgcc agccacgacg gcggcaagca ggccctggag 3540
   accgtgcaga ggctgctgcc tgtgctgtgc caggcccacg gcctgaccgg tcagcaggtg 3600
   gtggccatcg ccagccacga cggcggcaag caggccctgg agaccgtgca gaggctgctg 3660
   cctgtgctgt gccaggccca cggcctgacc ggtgagcagg tggtggccat cgccagcaac 3720
   atcggcggca agcaggccct ggagaccgtg cagaggctgc tgcctgtgct gtgccaggcc 3780
   cacggcctga ccggtcagca ggtggtggcc atcgccagca acaacggcgg caagcaggcc 3840
   ctggagaccg tgcagaggct gctgcctgtg ctgtgccagg cccacggcct gaccggtcag 3900
   caggtggtgg ccatcgccag caacggcggc ggcaagcagg ccctggagac cgtgcagagg 3960
   ctgctgcctg tgctgtgcca ggcccacggc ctgaccggtc agcaggtggt ggccatcgcc 4020
   agccacgacg gcggcaagca ggccctggag accgtgcaga ggctgctgcc tgtgctgtgc 4080
   caggcccacg gcctgaccgg tcagcaggtg gtggccatcg ccagcaacgg cggcggcagg 4140
   cctgccctgg agagcatcgt ggcccagctg agcaggcctg accctgccct ggccggatcc 4200
   ggcggcggcg gcggcggcgg ccaactagtc aaaagtgaac tggaggagaa gaaatctgaa 4260
   cttcgtcata aattgaaata tgtgcctcat gaatatattg aattaattga aattgccaga 4320
   aattccactc aggatagaat tcttgaaatg aaggtaatgg aattttttat gaaagtttat 4380
   ggatatagag gtaaacattt gggtggatca aggaaaccgg acggagcaat ttatactgtc 4440
   ggatctccta ttgattacgg tgtgatcgtg gatactaaag cttatagcgg aggttataat 4500
   ctgccaattg gccaagcaga tgaaatgcaa cgatatgtca aagaaaatca aacacgaaac 4560
   aaacatatca accctaatga atggtggaaa gtctatccat cttctgtaac ggaatttaag 4620
   tttttatttg tgagtggtca ctttaaagga aactacaaag ctcagcttac acgattaaat 4680
   cataagacta attgtaatgg agctgttctt agtgtagaag agcttttaat tggtggagaa 4740
   atgattaaag ccggcacatt aaccttagag gaagtgagac ggaaatttaa taacggcgag 4800
   ataaactttg ctagcggatc cacgcgtaaa tgattgcaga tccactagtt ctagagctcg 4860
   ctgatcagcc tcgactgtgc cttctagttg ccagccatct gttgtttgcc cctcccccgt 4920
   gccttccttg accctggaag gtgccactcc cactgtcctt tcctaataaa atgaggaaat 4980
   tgcatcgcat tgtctgagta ggtgtcattc tattctgggg ggtggggtgg ggcaggacag 5040
   caagggggag gattgggaag acaatagcag gcatgctggg gatgcggtgg gctctatggc 5100
   ttctgaggcg gaaagaacca gctggggctc gagatccact agttctagcc tcgaggctag 5160
   agcggccgcc accgcggtgg agctccaatt cgccctatag tgagtcgtat tacgcgcgct 5220
   cactggccgt cgttttacaa cgtcgtgact gggaaaaccc tggcgttacc caacttaatc 5280
   gccttgcagc acatccccct ttcgccagct ggcgtaatag cgaagaggcc cgcaccgatc 5340
   gcccttccca acagttgcgc agcctgaatg gcgaatggga cgcgccctgt agcggcgcat 5400
   taagcgcggc gggtgtggtg gttacgcgca gcgtgaccgc tacacttgcc agcgccctag 5460
   cgcccgctcc tttcgctttc ttcccttcct ttctcgccac gttcgccggc tttccccgtc 5520
   aagctctaaa tcgggggctc cctttagggt tccgatttag tgctttacgg cacctcgacc 5580
   ccaaaaaact tgattagggt gatggttcac gtagtgggcc atcgccctga tagacggttt 5640
   ttcgcccttt gacgttggag tccacgttct ttaatagtgg actcttgttc caaactggaa 5700
   caacactcaa ccctatctcg gtctattctt ttgatttata agggattttg ccgatttcgg 5760
   cctattggtt aaaaaatgag ctgatttaac aaaaatttaa cgcgaatttt aacaaaatat 5820
   taacgcttac aatttaggtg gcacttttcg gggaaatgtg cgcggaaccc ctatttgttt 5880
   atttttctaa atacattcaa atatgtatcc gctcatgaga caataaccct gataaatgct 5940
   tcaataatat tgaaaaagga agagtatgag tattcaacat ttccgtgtcg cccttattcc 6000
   cttttttgcg gcattttgcc ttcctgtttt tgctcaccca gaaacgctgg tgaaagtaaa 6060
   agatgctgaa gatcagttgg gtgcacgagt gggttacatc gaactggatc tcaacagcgg 6120
   taagatcctt gagagttttc gccccgaaga acgttttcca atgatgagca cttttaaagt 6180
   tctgctatgt ggcgcggtat tatcccgtat tgacgccggg caagagcaac tcggtcgccg 6240
   catacactat tctcagaatg acttggttga gtactcacca gtcacagaaa agcatcttac 6300
   ggatggcatg acagtaagag aattatgcag tgctgccata accatgagtg ataacactgc 6360
   ggccaactta cttctgacaa cgatcggagg accgaaggag ctaaccgctt ttttgcacaa 6420
   catgggggat catgtaactc gccttgatcg ttgggaaccg gagctgaatg aagccatacc 6480
   aaacgacgag cgtgacacca cgatgcctgt agcaatggca acaacgttgc gcaaactatt 6540
   aactggcgaa ctacttactc tagcttcccg gcaacaatta atagactgga tggaggcgga 6600
   taaagttgca ggaccacttc tgcgctcggc ccttccggct ggctggttta ttgctgataa 6660
   atctggagcc ggtgagcgtg ggtctcgcgg tatcattgca gcactggggc cagatggtaa 6720
   gccctcccgt atcgtagtta tctacacgac ggggagtcag gcaactatgg atgaacgaaa 6780
   tagacagatc gctgagatag gtgcctcact gattaagcat tggtaactgt cagaccaagt 6840
   ttactcatat atactttaga ttgatttaaa acttcatttt taatttaaaa ggatctaggt 6900
   gaagatcctt tttgataatc tcatgaccaa aatcccttaa cgtgagtttt cgttccactg 6960
   agcgtcagac cccgtagaaa agatcaaagg atcttcttga gatccttttt ttctgcgcgt 7020
   aatctgctgc ttgcaaacaa aaaaaccacc gctaccagcg gtggtttgtt tgccggatca 7080
   agagctacca actctttttc cgaaggtaac tggcttcagc agagcgcaga taccaaatac 7140
   tgtccttcta gtgtagccgt agttaggcca ccacttcaag aactctgtag caccgcctac 7200
   atacctcgct ctgctaatcc tgttaccagt ggctgctgcc agtggcgata agtcgtgtct 7260
   taccgggttg gactcaagac gatagttacc ggataaggcg cagcggtcgg gctgaacggg    7320
   gggttcgtgc acacagccca gcttggagcg aacgacctac accgaactga gatacctaca    7380
   gcgtgagcta tgagaaagcg ccacgcttcc cgaagggaga aaggcggaca ggtatccggt    7440
   aagcggcagg gtcggaacag gagagcgcac gagggagctt ccagggggaa acgcctggta    7500
   tctttatagt cctgtcgggt ttcgccacct ctgacttgag cgtcgatttt tgtgatgctc    7560
   gtcagggggg cggagcctat ggaaaaacgc cagcaacgcg gcctttttac ggttcctggc    7620
   cttttgctgg ccttttgctc acatgttctt tcctgcgtta tcccctgatt ctgtggataa    7680
   ccgtattacc gcctttgagt gagctgatac cgctcgccgc agccgaacga ccgagcgcag    7740
   cgagtcagtg agcgaggaag cggaagagcg cccaatacgc aaaccgcctc tccccgcgcg    7800
   ttggccgatt cattaatgca gctggcacga caggtttccc gactggaaag cgggcagtga    7860
   gcgcaacgca attaatgtga gttagctcac tcattaggca ccccaggctt tacactttat    7920
   gcttccggct cgtatgttgt gtggaattgt gagcggataa caatttcaca caggaaacag    7980
   ctatgaccat gattacgcca agcgcgcaat taaccctcac taaagggaac aaaagct       8037
<210> 5
   <211> 1012
   <212> PRT
   <213> Artificial Sequence
<220>
   <223> /note="Description of artificial sequence: Aminoacid sequence of protein
   venus-TalRosa2-Fok-KK"
<400> 5
   Met Pro Lys Lys Lys Arg Lys Val Met Val Ser Lys Gly Glu Glu Leu
   1               5                   10                  15
   Phe Thr Gly Val Val Pro Ile Leu Val Glu Leu Asp Gly Asp Val Asn
               20                  25                  30
   Gly His Lys Phe Ser Val Ser Gly Glu Gly Glu Gly Asp Ala Thr Tyr
           35                  40                  45
   Gly Lys Leu Thr Leu Lys Leu Ile Cys Thr Thr Gly Lys Leu Pro Val
       50                  55                  60
   Pro Trp Pro Thr Leu Val Thr Thr Leu Gly Tyr Gly Leu Gln Cys Phe
   65                  70                  75                  80
   Ala Arg Tyr Pro Asp His Met Lys Gln His Asp Phe Phe Lys Ser Ala
                   85                  90                  95
   Met Pro Glu Gly Tyr Val Gln Glu Arg Thr Ile Phe Phe Lys Asp Asp
               100                 105                 110
   Gly Asn Tyr Lys Thr Arg Ala Glu Val Lys Phe Glu Gly Asp Thr Leu
           115                 120                 125
   Val Asn Arg Ile Glu Leu Lys Gly Ile Asp Phe Lys Glu Asp Gly Asn
       130                 135                 140
   Ile Leu Gly His Lys Leu Glu Tyr Asn Tyr Asn Ser His Asn Val Tyr
   145                 150                 155                 160
   Ile Thr Ala Asp Lys Gln Lys Asn Gly Ile Lys Ala Asn Phe Lys Ile
                   165                 170                 175
   Arg His Asn Ile Glu Asp Gly Gly Val Gln Leu Ala Asp His Tyr Gln
               180                 185                 190
   Gln Asn Thr Pro Ile Gly Asp Gly Pro Val Leu Leu Pro Asp Asn His
           195                 200                 205
   Tyr Leu Ser Tyr Gln Ser Ala Leu Ser Lys Asp Pro Asn Glu Lys Arg
       210                 215                 220
   Asp His Met Val Leu Leu Glu Phe Val Thr Ala Ala Gly Ile Thr Leu
   225                 230                 235                 240
   Gly Met Asp Glu Leu Tyr Lys Gly Gly Gly Gly Gly Gly Gly Thr Arg
                   245                 250                 255
   Leu Asp Thr Gly Gln Leu Leu Lys Ile Ala Lys Arg Gly Gly Val Thr
               260                 265                 270
   Ala Val Glu Ala Val His Ala Trp Arg Asn Ala Leu Thr Gly Ala Pro
           275                 280                 285
   Leu Asn Leu Thr Gly Gln Gln Val Val Ala Ile Ala Ser His Asp Gly
       290                 295                 300
   Gly Lys Gln Ala Leu Glu Thr Val Gln Arg Leu Leu Pro Val Leu Cys
   305                 310                 315                 320
   Gln Ala His Gly Leu Thr Gly Gln Gln Val Val Ala Ile Ala Ser Asn
                   325                 330                 335
   Gly Gly Gly Lys Gln Ala Leu Glu Thr Val Gln Arg Leu Leu Pro Val
               340                 345                 350
   Leu Cys Gln Ala His Gly Leu Thr Gly Gln Gln Val Val Ala Ile Ala
           355                 360                 365
   Ser Asn Asn Gly Gly Lys Gln Ala Leu Glu Thr Val Gln Arg Leu Leu
       370                 375                 380
   Pro Val Leu Cys Gln Ala His Gly Leu Thr Gly Gln Gln Val Val Ala
   385                 390                 395                 400
   Ile Ala Ser His Asp Gly Gly Lys Gln Ala Leu Glu Thr Val Gln Arg
                   405                 410                 415
   Leu Leu Pro Val Leu Cys Gln Ala His Gly Leu Thr Gly Glu Gln Val
               420                 425                 430
   Val Ala Ile Ala Ser Asn Ile Gly Gly Lys Gln Ala Leu Glu Thr Val
           435                 440                 445
   Gln Arg Leu Leu Pro Val Leu Cys Gln Ala His Gly Leu Thr Gly Glu
       450                 455                 460
   Gln Val Val Ala Ile Ala Ser Asn Ile Gly Gly Lys Gln Ala Leu Glu
   465                 470                 475                 480
   Thr Val Gln Arg Leu Leu Pro Val Leu Cys Gln Ala His Gly Leu Thr
                   485                 490                 495
   Gly Gln Gln Val Val Ala Ile Ala Ser His Asp Gly Gly Lys Gln Ala
               500                 505                 510
   Leu Glu Thr Val Gln Arg Leu Leu Pro Val Leu Cys Gln Ala His Gly
           515                 520                 525
   Leu Thr Gly Gln Gln Val Val Ala Ile Ala Ser Asn Gly Gly Gly Lys
       530                 535                 540
   Gln Ala Leu Glu Thr Val Gln Arg Leu Leu Pro Val Leu Cys Gln Ala
   545                 550                 555                 560
   His Gly Leu Thr Gly Gln Gln Val Val Ala Ile Ala Ser His Asp Gly
                   565                 570                 575
   Gly Lys Gln Ala Leu Glu Thr Val Gln Arg Leu Leu Pro Val Leu Cys
               580                 585                 590
   Gln Ala His Gly Leu Thr Gly Gln Gln Val Val Ala Ile Ala Ser His
           595                 600                 605
   Asp Gly Gly Lys Gln Ala Leu Glu Thr Val Gln Arg Leu Leu Pro Val
       610                 615                 620
   Leu Cys Gln Ala His Gly Leu Thr Gly Glu Gln Val Val Ala Ile Ala
   625                 630                 635                 640
   Ser Asn Ile Gly Gly Lys Gln Ala Leu Glu Thr Val Gln Arg Leu Leu
                   645                 650                 655
   Pro Val Leu Cys Gln Ala His Gly Leu Thr Gly Gln Gln Val Val Ala
               660                 665                 670
   Ile Ala Ser Asn Asn Gly Gly Lys Gln Ala Leu Glu Thr Val Gln Arg
           675                 680                 685
   Leu Leu Pro Val Leu Cys Gln Ala His Gly Leu Thr Gly Gln Gln Val
       690                 695                 700
   Val Ala Ile Ala Ser Asn Gly Gly Gly Lys Gln Ala Leu Glu Thr Val
   705                 710                 715                 720
   Gln Arg Leu Leu Pro Val Leu Cys Gln Ala His Gly Leu Thr Gly Gln
                   725                 730                 735
   Gln Val Val Ala Ile Ala Ser His Asp Gly Gly Lys Gln Ala Leu Glu
               740                 745                 750
   Thr Val Gln Arg Leu Leu Pro Val Leu Cys Gln Ala His Gly Leu Thr
           755                 760                 765
   Gly Gln Gln Val Val Ala Ile Ala Ser Asn Gly Gly Gly Arg Pro Ala
       770                 775                 780
   Leu Glu Ser Ile Val Ala Gln Leu Ser Arg Pro Asp Pro Ala Leu Ala
   785                 790                 795                 800
   Gly Ser Gly Gly Gly Gly Gly Gly Gly Gln Leu Val Lys Ser Glu Leu
                   805                 810                 815
   Glu Glu Lys Lys Ser Glu Leu Arg His Lys Leu Lys Tyr Val Pro His
               820                 825                 830
   Glu Tyr Ile Glu Leu Ile Glu Ile Ala Arg Asn Ser Thr Gln Asp Arg
           835                 840                 845
   Ile Leu Glu Met Lys Val Met Glu Phe Phe Met Lys Val Tyr Gly Tyr
       850                 855                 860
   Arg Gly Lys His Leu Gly Gly Ser Arg Lys Pro Asp Gly Ala Ile Tyr
   865                 870                 875                 880
   Thr Val Gly Ser Pro Ile Asp Tyr Gly Val Ile Val Asp Thr Lys Ala
                   885                 890                 895
   Tyr Ser Gly Gly Tyr Asn Leu Pro Ile Gly Gln Ala Asp Glu Met Gln
               900                 905                 910
   Arg Tyr Val Lys Glu Asn Gln Thr Arg Asn Lys His Ile Asn Pro Asn
           915                 920                 925
   Glu Trp Trp Lys Val Tyr Pro Ser Ser Val Thr Glu Phe Lys Phe Leu
       930                 935                 940
   Phe Val Ser Gly His Phe Lys Gly Asn Tyr Lys Ala Gln Leu Thr Arg
   945                 950                 955                 960
   Leu Asn His Lys Thr Asn Cys Asn Gly Ala Val Leu Ser Val Glu Glu
                   965                 970                 975
   Leu Leu Ile Gly Gly Glu Met Ile Lys Ala Gly Thr Leu Thr Leu Glu
               980                 985                 990
   Glu Val Arg Arg Lys Phe Asn Asn  Gly Glu Ile Asn Phe  Ala Ser Gly
           995                 1000                 1005
   Ser Thr  Arg Lys
       1010
<210> 6
   <211> 12565
   <212> DNA
   <213> Artificial Sequence
<220>
   <223> /note="Description of artificial sequence: gene targeting vector
   pRosa26.8-2"
<400> 6
   caccgcatta ccctgttatc cctagcggca ggccctccga gcgtggtgga gccgttctgt      60
   gagacagccg ggtacgagtc gtgacgctgg aaggggcaag cgggtggtgg gcaggaatgc     120
   ggtccgccct gcagcaaccg gagggggagg gagaagggag cggaaaagtc tccaccggac     180
   gcggccatgg ctcggggggg ggggggcagc ggaggagcgc ttccggccga cgtctcgtcg     240
   ctgattggct tcttttcctc ccgccgtgtg tgaaaacaca aatggcgtgt tttggttggc     300
   gtaaggcgcc tgtcagttaa cggcagccgg agtgcgcagc cgccggcagc ctcgctctgc     360
   ccactgggtg gggcgggagg taggtggggt gaggcgagct ggacgtgcgg gcgcggtcgg     420
   cctctggcgg ggcgggggag gggagggagg gtcagcgaaa gtagctcgcg cgcgagcggc     480
   cgcccaccct ccccttcctc tgggggagtc gttttacccg ccgccggccg ggcctcgtcg     540
   tctgattggc tctcggggcc cagaaaactg gcccttgcca ttggctcgtg ttcgtgcaag     600
   ttgagtccat ccgccggcca gcgggggcgg cgaggaggcg ctcccaggtt ccggccctcc     660
   cctcggcccc gcgccgcaga gtctggccgc gcgcccctgc gcaacgtggc aggaagcgcg     720
   cgctgggggc ggggacgggc agtagggctg agcggctgcg ggcgggtgcc aagcacgttt      780
   ccgacttgag ttgcctcaag aggggcgtgc tgagccagac ctccatcgcg cactccgggg     840
   agtggaggga aggagcgagg gctcagttgg gctgttttgg aggcaggaag cacttgctct     900
   cccaaagtcg ctctgagttg ttatcagtaa gggagctgca gtggagtagg cggggagaag     960
   gccgcaccct tctccggagg ggggagggga gtgttgcaat acctttctgg gagttctctg    1020
   ctgcctcctg gcttctgagg accgccctgg gcctgggaga atcccttccc cctcttccct    1080
   cgtgatctgc aactccagtc tttctaggcg cgccctcgag gtgacctgca cgtctagggc 1140
   gcagtagtcc agggtttcct tgatgatgtc atacttatcc tgtccctttt ttttccacag 1200
   ctcgcggttg aggacaaact cttcgcggtc tttccagtac taggggatcg aaagagcctg 1260
   ctaaagcaaa aaagaagtca ccatgtcgtt tactttgacc aacaagaacg tgattttcgt 1320
   tgccggtctg ggaggcattg gtctggacac cagcaaggag ctgctcaagc gcgatcccgt 1380
   cgttttacaa cgtcgtgact gggaaaaccc tggcgttacc caacttaatc gccttgcagc 1440
   acatccccct ttcgccagct ggcgtaatag cgaagaggcc cgcaccgatc gcccttccca 1500
   acagttgcgc agcctgaatg gcgaatggcg ctttgcctgg tttccggcac cagaagcggt 1560
   gccggaaagc tggctggagt gcgatcttcc tgaggccgat actgtcgtcg tcccctcaaa 1620
   ctggcagatg cacggttacg atgcgcccat ctacaccaac gtgacctatc ccattacggt 1680
   caatccgccg tttgttccca cggagaatcc gacgggttgt tactcgctca catttaatgt 1740
   tgatgaaagc tggctacagg aaggccagac gcgaattatt tttgatggcg ttaactcggc 1800
   gtttcatctg tggtgcaacg ggcgctgggt cggttacggc caggacagtc gtttgccgtc 1860
   tgaatttgac ctgagcgcat ttttacgcgc cggagaaaac cgcctcgcgg tgatggtgct 1920
   gcgctggagt gacggcagtt atctggaaga tcaggatatg tggcggatga gcggcatttt 1980
   ccgtgacgtc tcgttgctgc ataaaccgac tacacaaatc agcgatttcc atgttgccac 2040
   tcgctttaat gatgatttca gccgcgctgt actggaggct gaagttcaga tgtgcggcga 2100
   gttgcgtgac tacctacggg taacagtttc tttatggcag ggtgaaacgc aggtcgccag 2160
   cggcaccgcg cctttcggcg gtgaaattat cgatgagcgt ggtggttatg ccgatcgcgt 2220
   cacactacgt ctgaacgtcg aaaacccgaa actgtggagc gccgaaatcc cgaatctcta 2280
   tcgtgcggtg gttgaactgc acaccgccga cggcacgctg attgaagcag aagcctgcga 2340
   tgtcggtttc cgcgaggtgc ggattgaaaa tggtctgctg ctgctgaacg gcaagccgtt 2400
   gctgattcga ggcgttaacc gtcacgagca tcatcctctg catggtcagg tcatggatga 2460
   gcagacgatg gtgcaggata tcctgctgat gaagcagaac aactttaacg ccgtgcgctg 2520
   ttcgcattat ccgaaccatc cgctgtggta cacgctgtgc gaccgctacg gcctgtatgt 2580
   ggtggatgaa gccaatattg aaacccacgg catggtgcca atgaatcgtc tgaccgatga 2640
   tccgcgctgg ctaccggcga tgagcgaacg cgtaacgcga atggtgcagc gcgatcgtaa 2700
   tcacccgagt gtgatcatct ggtcgctggg gaatgaatca ggccacggcg ctaatcacga 2760
   cgcgctgtat cgctggatca aatctgtcga tccttcccgc ccggtgcagt atgaaggcgg 2820
   cggagccgac accacggcca ccgatattat ttgcccgatg tacgcgcgcg tggatgaaga 2880
   ccagcccttc ccggctgtgc cgaaatggtc catcaaaaaa tggctttcgc tacctggaga 2940
   gacgcgcccg ctgatccttt gcgaatacgc ccacgcgatg ggtaacagtc ttggcggttt 3000
   cgctaaatac tggcaggcgt ttcgtcagta tccccgttta cagggcggct tcgtctggga 3060
   ctgggtggat cagtcgctga ttaaatatga tgaaaacggc aacccgtggt cggcttacgg 3120
   cggtgatttt ggcgatacgc cgaacgatcg ccagttctgt atgaacggtc tggtctttgc 3180
   cgaccgcacg ccgcatccag cgctgacgga agcaaaacac cagcagcagt ttttccagtt 3240
   ccgtttatcc gggcaaacca tcgaagtgac cagcgaatac ctgttccgtc atagcgataa 3300
   cgagctcctg cactggatgg tggcgctgga tggtaagccg ctggcaagcg gtgaagtgcc 3360
   tctggatgtc gctccacaag gtaaacagtt gattgaactg cctgaactac cgcagccgga 3420
   gagcgccggg caactctggc tcacagtacg cgtagtgcaa ccgaacgcga ccgcatggtc 3480
   agaagccggg cacatcagcg cctggcagca gtggcgtctg gcggaaaacc tcagtgtgac 3540
   gctccccgcc gcgtcccacg ccatcccgca tctgaccacc agcgaaatgg atttttgcat 3600
   cgagctgggt aataagcgtt ggcaatttaa ccgccagtca ggctttcttt cacagatgtg 3660
   gattggcgat aaaaaacaac tgctgacgcc gctgcgcgat cagttcaccc gtgcaccgct 3720
   ggataacgac attggcgtaa gtgaagcgac ccgcattgac cctaacgcct gggtcgaacg 3780
   ctggaaggcg gcgggccatt accaggccga agcagcgttg ttgcagtgca cggcagatac 3840
   acttgctgat gcggtgctga ttacgaccgc tcacgcgtgg cagcatcagg ggaaaacctt 3900
   atttatcagc cggaaaacct accggattga tggtagtggt caaatggcga ttaccgttga 3960
   tgttgaagtg gcgagcgata caccgcatcc ggcgcggatt ggcctgaact gccagctggc 4020
   gcaggtagca gagcgggtaa actggctcgg attagggccg caagaaaact atcccgaccg 4080
   ccttactgcc gcctgttttg accgctggga tctgccattg tcagacatgt ataccccgta 4140
   cgtcttcccg agcgaaaacg gtctgcgctg cgggacgcgc gaattgaatt atggcccaca 4200
   ccagtggcgc ggcgacttcc agttcaacat cagccgctac agtcaacagc aactgatgga 4260
   aaccagccat cgccatctgc tgcacgcgga agaaggcaca tggctgaata tcgacggttt 4320
   ccatatgggg attggtggcg acgactcctg gagcccgtca gtatcggcgg aattacagct 4380
   gagcgccggt cgctaccatt accagttggt ctggtgtcaa aaataataat aaccgggcag 4440
   gccatgtctg cccgtatttc gcgtaaggaa atccattatg tactatttaa aaaacacaaa 4500
   cttttggatg ttcggtttat tctttttctt ttactttttt atcatgggag cctacttccc 4560
   gtttttcccg atttggctac atgacatcaa ccatatcagc aaaagtgata cgggtattat 4620
   ttttgccgct atttctctgt tctcgctatt attccaaccg ctgtttggtc tgctttctga 4680
   caaactcggc ctcgactcta ggcggccgcg gggatccaga catgataaga tacattgatg 4740
   agtttggaca aaccacaact agaatgcagt gaaaaaaatg ctttatttgt gaaatttgtg 4800
   atgctattgc tttatttgta accattataa gctgcaataa acaagttaac aacaacaatt 4860
   gcattcattt tatgtttcag gttcaggggg aggtgtggga ggttttttcg gatcctctag 4920
   agtcgagggc tgcagatctg tagggcgcag tagtccaggg tttccttgat gatgtcatac 4980
   ttatcctgtc cctttttttt ccacagctcg cggttgagga caaactcttc gcggtctttc 5040
   cagtggggat cgacggtatc gataagctgg ccgctctagt ggccgtacgg gcccacctgc 5100
   cgggccactt aattaaattt aaatcacgtg ctagcgctta agcttgaagt tcctattccg 5160
   aagttcctat tctctagaaa gtataggaac ttcggcgcgc cgtcgacgtt taaacatgca 5220
   tgaagttcct attccgaagt tcctattctc tagaaagtat aggaacttca taaaacctgc 5280
   aggcatgcaa gcgatcgcgg ccggccaagg cccgcggggc cactagaaga tgggcgggag 5340
   tcttctgggc aggcttaaag gctaacctgg tgtgtgggcg ttgtcctgca ggggaattga 5400
   acaggtgtaa aattggaggg acaagacttc ccacagattt tcggttttgt cgggaagttt 5460
   tttaataggg gcaaataagg aaaatgggag gataggtagt catctggggt tttatgcagc 5520
   aaaactacag gttattattg cttgtgatcc gcctcggagt attttccatc gaggtagatt 5580
   aaagacatgc tcacccgagt tttatactct cctgcttgag atccttacta cagtatgaaa 5640
   ttacagtgtc gcgagttaga ctatgtaagc agaattttaa tcatttttaa agagcccagt 5700
   acttcatatc catttctccc gctccttctg cagccttatc aaaaggtatt ttagaacact 5760
   cattttagcc ccattttcat ttattatact ggcttatcca acccctagac agagcattgg 5820
   cattttccct ttcctgatct tagaagtctg atgactcatg aaaccagaca gattagttac 5880
   atacaccaca aatcgaggct gtagctgggg cctcaacact gcagttcttt tataactcct 5940
   tagtacactt tttgttgatc ctttgccttg atccttaatt ttcagtgtct atcacctctc 6000
   ccgtcaggtg gtgttccaca tttgggccta ttctcagtcc agggagtttt acaacaatag 6060
   atgtattgag aatccaacct aaagcttaac tttccactcc catgaatgcc tctctccttt 6120
   ttctccattt ataaactgag ctattaacca ttaatggttt ccaggtggat gtctcctccc 6180
   ccaatattac ctgatgtatc ttacatattg ccaggctgat attttaagac attaaaaggt 6240
   atatttcatt attgagccac atggtattga ttactgctta ctaaaatttt gtcattgtac 6300
   acatctgtaa aaggtggttc cttttggaat gcaaagttca ggtgtttgtt gtctttcctg 6360
   acctaaggtc ttgtgagctt gtattttttc tatttaagca gtgctttctc ttggactggc 6420
   ttgactcatg gcattctaca cgttattgct ggtctaaatg tgattttgcc aagcttcttc 6480
   aggacctata attttgcttg acttgtagcc aaacacaagt aaaatgatta agcaacaaat 6540
   gtatttgtga agcttggttt ttaggttgtt gtgttgtgtg tgcttgtgct ctataataat 6600
   actatccagg ggctggagag gtggctcgga gttcaagagc acagactgct cttccagaag 6660
   tcctgagttc aattcccagc aaccacatgg tggctcacaa ccatctgtaa tgggatctga 6720
   tgccctcttc tggtgtgtct gaagaccaca agtgtattca cattaaataa ataaatcctc 6780
   cttcttcttc tttttttttt ttttaaagag aatactgtct ccagtagaat ttactgaagt 6840
   aatgaaatac tttgtgtttg ttccaatatg gtagccaata atcaaattac tctttaagca 6900
   ctggaaatgt taccaaggaa ctaattttta tttgaagtgt aactgtggac agaggagcca 6960
   taactgcaga cttgtgggat acagaagacc aatgcagact ttaatgtctt ttctcttaca 7020
   ctaagcaata aagaaataaa aattgaactt ctagtatcct atttgtttaa actgctagct 7080
   ttacttaact tttgtgcttc atctatacaa agctgaaagc taagtctgca gccattacta 7140
   aacatgaaag caagtaatga taattttgga tttcaaaaat gtagggccag agtttagcca 7200
   gccagtggtg gtgcttgcct ttatgccttt aatcccagca ctctggaggc agagacaggc 7260
   agatctctga gtttgagccc agcctggtct acacatcaag ttctatctag gatagccagg 7320
   aatacacaca gaaaccctgt tggggagggg ggctctgaga tttcataaaa ttataattga 7380
   agcattccct aatgagccac tatggatgtg gctaaatccg tctacctttc tgatgagatt 7440
   tgggtattat tttttctgtc tctgctgttg gttgggtctt ttgacactgt gggctttctt 7500
   taaagcctcc ttcctgccat gtggtctctt gtttgctact aacttcccat ggcttaaatg 7560
   gcatggcttt ttgccttcta agggcagctg ctgagatttg cagcctgatt tccagggtgg 7620
   ggttgggaaa tctttcaaac actaaaattg tcctttaatt ttttttttaa aaaatgggtt 7680
   atataataaa cctcataaaa tagttatgag gagtgaggtg gactaatatt aaatgagtcc 7740
   ctcccctata aaagagctat taaggctttt tgtcttatac ttaacttttt ttttaaatgt 7800
   ggtatcttta gaaccaaggg tcttagagtt ttagtataca gaaactgttg catcgcttaa 7860
   tcagattttc tagtttcaaa tccagagaat ccaaattctt cacagccaaa gtcaaattaa 7920
   gaatttctga cttttaatgt taatttgctt actgtgaata taaaaatgat agcttttcct 7980
   gaggcagggt ctcactatgt atctctgcct gatctgcaac aagatatgta gactaaagtt 8040
   ctgcctgctt ttgtctcctg aatactaagg ttaaaatgta gtaatacttt tggaacttgc 8100
   aggtcagatt cttttatagg ggacacacta agggagcttg ggtgatagtt ggtaaaatgt 8160
   gtttcaagtg atgaaaactt gaattattat caccgcaacc tactttttaa aaaaaaaagc 8220
   caggcctgtt agagcatgct taagggatcc ctaggacttg ctgagcacac aagagtagtt 8280
   acttggcagg ctcctggtga gagcatattt caaaaaacaa ggcagacaac caagaaacta 8340
   cagttaaggt tacctgtctt taaaccatct gcatatacac agggatatta aaatattcca 8400
   aataatattt cattcaagtt ttcccccatc aaattgggac atggatttct ccggtgaata 8460
   ggcagagttg gaaactaaac aaatgttggt tttgtgattt gtgaaattgt tttcaagtga 8520
   tagttaaagc ccatgagata cagaacaaag ctgctatttc gaggtctctt ggtttatact 8580
   cagaagcact tctttgggtt tccctgcact atcctgatca tgtgctaggc ctaccttagg 8640
   ctgattgttg ttcaaataaa cttaagtttc ctgtcaggtg atgtcatatg atttcatata 8700
   tcaaggcaaa acatgttata tatgttaaac atttgtactt aatgtgaaag ttaggtcttt 8760
   gtgggtttga tttttaattt tcaaaacctg agctaaataa gtcattttta catgtcttac 8820
   atttggtgga attgtataat tgtggtttgc aggcaagact ctctgaccta gtaaccctac 8880
   ctatagagca ctttgctggg tcacaagtct aggagtcaag catttcacct tgaagttgag 8940
   acgttttgtt agtgtatact agtttatatg ttggaggaca tgtttatcca gaagatattc 9000
   aggactattt ttgactgggc taaggaattg attctgatta gcactgttag tgagcattga 9060
   gtggccttta ggcttgaatt ggagtcactt gtatatctca aataatgctg gcctttttta 9120
   aaaagccctt gttctttatc accctgtttt ctacataatt tttgttcaaa gaaatacttg 9180
   tttggatctc cttttgacaa caatagcatg ttttcaagcc atattttttt tccttttttt 9240
   tttttttttt ggtttttcga gacagggttt ctctgtatag ccctggctgt cctggaactc 9300
   actttgtaga ccaggctggc ctcgaactca gaaatccgcc tgcctctgcc tcctgagtgc 9360
   cgggattaaa ggcgtgcacc accacgcctg gctaagttgg atattttgtt atataactat 9420
   aaccaatact aactccactg ggtggatttt taattcagtc agtagtctta agtggtcttt 9480
   attggccctt cattaaaatc tactgttcac tctaacagag gctgttggta ctagtggcac 9540
   ttaagcaact tcctacggat atactagcag attaagggtc agggatagaa actagtctag 9600
   cgttttgtat acctaccagc tttatactac cttgttctga tagaaatatt tcaggacatc 9660
   tagcttatcg atccgtcgac ggtatcgata agcttgatat cgaattccag cttttgttcc 9720
   ctttagtgag ggttaattgc gcgcttggcg taatcatggt catagctgtt tcctgtgtga 9780
   aattgttatc cgctcacaat tccacacaac atacgagccg gaagcataaa gtgtaaagcc 9840
   tggggtgcct aatgagtgag ctaactcaca ttaattgcgt tgcgctcact gcccgctttc 9900
   cagtcgggaa acctgtcgtg ccagctgcat taatgaatcg gccaacgcgc ggggagaggc 9960
   ggtttgcgta ttgggcgctc ttccgcttcc tcgctcactg actcgctgcg ctcggtcgtt 10020
   cggctgcggc gagcggtatc agctcactca aaggcggtaa tacggttatc cacagaatca 10080
   ggggataacg caggaaagaa catgtgagca aaaggccagc aaaaggccag gaaccgtaaa 10140
   aaggccgcgt tgctggcgtt tttccatagg ctccgccccc ctgacgagca tcacaaaaat 10200
   cgacgctcaa gtcagaggtg gcgaaacccg acaggactat aaagatacca ggcgtttccc 10260
   cctggaagct ccctcgtgcg ctctcctgtt ccgaccctgc cgcttaccgg atacctgtcc 10320
   gcctttctcc cttcgggaag cgtggcgctt tctcatagct cacgctgtag gtatctcagt 10380
   tcggtgtagg tcgttcgctc caagctgggc tgtgtgcacg aaccccccgt tcagcccgac 10440
   cgctgcgcct tatccggtaa ctatcgtctt gagtccaacc cggtaagaca cgacttatcg 10500
   ccactggcag cagccactgg taacaggatt agcagagcga ggtatgtagg cggtgctaca 10560
   gagttcttga agtggtggcc taactacggc tacactagaa ggacagtatt tggtatctgc 10620
   gctctgctga agccagttac cttcggaaaa agagttggta gctcttgatc cggcaaacaa 10680
   accaccgctg gtagcggtgg tttttttgtt tgcaagcagc agattacgcg cagaaaaaaa 10740
   ggatctcaag aagatccttt gatcttttct acggggtctg acgctcagtg gaacgaaaac 10800
   tcacgttaag ggattttggt catgagatta tcaaaaagga tcttcaccta gatcctttta 10860
   aattaaaaat gaagttttaa atcaatctaa agtatatatg agtaaacttg gtctgacagt 10920
   taccaatgct taatcagtga ggcacctatc tcagcgatct gtctatttcg ttcatccata 10980
   gttgcctgac tccccgtcgt gtagataact acgatacggg agggcttacc atctggcccc 11040
   agtgctgcaa tgataccgcg agacccacgc tcaccggctc cagatttatc agcaataaac 11100
   cagccagccg gaagggccga gcgcagaagt ggtcctgcaa ctttatccgc ctccatccag 11160
   tctattaatt gttgccggga agctagagta agtagttcgc cagttaatag tttgcgcaac 11220
   gttgttgcca ttgctacagg catcgtggtg tcacgctcgt cgtttggtat ggcttcattc 11280
   agctccggtt cccaacgatc aaggcgagtt acatgatccc ccatgttgtg caaaaaagcg 11340
   gttagctcct tcggtcctcc gatcgttgtc agaagtaagt tggccgcagt gttatcactc 11400
   atggttatgg cagcactgca taattctctt actgtcatgc catccgtaag atgcttttct 11460
   gtgactggtg agtactcaac caagtcattc tgagaatagt gtatgcggcg accgagttgc 11520
   tcttgcccgg cgtcaatacg ggataatacc gcgccacata gcagaacttt aaaagtgctc 11580
   atcattggaa aacgttcttc ggggcgaaaa ctctcaagga tcttaccgct gttgagatcc 11640
   agttcgatgt aacccactcg tgcacccaac tgatcttcag catcttttac tttcaccagc 11700
   gtttctgggt gagcaaaaac aggaaggcaa aatgccgcaa aaaagggaat aagggcgaca 11760
   cggaaatgtt gaatactcat actcttcctt tttcaatatt attgaagcat ttatcagggt 11820
   tattgtctca tgagcggata catatttgaa tgtatttaga aaaataaaca aataggggtt 11880
   ccgcgcacat ttccccgaaa agtgccacct aaattgtaag cgttaatatt ttgttaaaat 11940
   tcgcgttaaa tttttgttaa atcagctcat tttttaacca ataggccgaa atcggcaaaa 12000
   tcccttataa atcaaaagaa tagaccgaga tagggttgag tgttgttcca gtttggaaca 12060
   agagtccact attaaagaac gtggactcca acgtcaaagg gcgaaaaacc gtctatcagg 12120
   gcgatggccc actacgtgaa ccatcaccct aatcaagttt tttggggtcg aggtgccgta 12180
   aagcactaaa tcggaaccct aaagggagcc cccgatttag agcttgacgg ggaaagccgg 12240
   cgaacgtggc gagaaaggaa gggaagaaag cgaaaggagc gggcgctagg gcgctggcaa 12300
   gtgtagcggt cacgctgcgc gtaaccacca cacccgccgc gcttaatgcg ccgctacagg 12360
   gcgcgtccca ttcgccattc aggctgcgca actgttggga agggcgatcg gtgcgggcct 12420
   cttcgctatt acgccagctg gcgaaagggg gatgtgctgc aaggcgatta agttgggtaa 12480
   cgccagggtt ttcccagtca cgacgttgta aaacgacggc cagtgagcgc gcgtaatacg 12540
   actcactata gggcgaattg gagct 12565
<210> 7
   <211> 3049
   <212> DNA
   <213> Artificial Sequence
<220>
   <223> /note="Description of artificial sequence: sequence of plasmid
   pbs-Rosa-targetseq"
<400> 7
   gctggaaaca tgcatgaagt tcctattccg aagttcctat tctctagaaa gtataggaac    60
   ttcataaaac ctgcaggcat gcaagcgatc gcggccggcc aaggcccgcg gggccactag   120
   ttctagagcg gcctgatctg caactccagt ctttctagaa gatgggcggg agtcttcggg   180
   ccgccaccgc ggtggagctc caattcgccc tatagtgagt cgtattacgc gcgctcactg   240
   gccgtcgttt tacaacgtcg tgactgggaa aaccctggcg ttacccaact taatcgcctt   300
   gcagcacatc cccctttcgc cagctggcgt aatagcgaag aggcccgcac cgatcgccct   360
   tcccaacagt tgcgcagcct gaatggcgaa tgggacgcgc cctgtagcgg cgcattaagc   420
   gcggcgggtg tggtggttac gcgcagcgtg accgctacac ttgccagcgc cctagcgccc 480
   gctcctttcg ctttcttccc ttcctttctc gccacgttcg ccggctttcc ccgtcaagct 540
   ctaaatcggg ggctcccttt agggttccga tttagtgctt tacggcacct cgaccccaaa 600
   aaacttgatt agggtgatgg ttcacgtagt gggccatcgc cctgatagac ggtttttcgc 660
   cctttgacgt tggagtccac gttctttaat agtggactct tgttccaaac tggaacaaca 720
   ctcaacccta tctcggtcta ttcttttgat ttataaggga ttttgccgat ttcggcctat 780
   tggttaaaaa atgagctgat ttaacaaaaa tttaacgcga attttaacaa aatattaacg 840
   cttacaattt aggtggcact tttcggggaa atgtgcgcgg aacccctatt tgtttatttt 900
   tctaaataca ttcaaatatg tatccgctca tgagacaata accctgataa atgcttcaat 960
   aatattgaaa aaggaagagt atgagtattc aacatttccg tgtcgccctt attccctttt 1020
   ttgcggcatt ttgccttcct gtttttgctc acccagaaac gctggtgaaa gtaaaagatg 1080
   ctgaagatca gttgggtgca cgagtgggtt acatcgaact ggatctcaac agcggtaaga 1140
   tccttgagag ttttcgcccc gaagaacgtt ttccaatgat gagcactttt aaagttctgc 1200
   tatgtggcgc ggtattatcc cgtattgacg ccgggcaaga gcaactcggt cgccgcatac 1260
   actattctca gaatgacttg gttgagtact caccagtcac agaaaagcat cttacggatg 1320
   gcatgacagt aagagaatta tgcagtgctg ccataaccat gagtgataac actgcggcca 1380
   acttacttct gacaacgatc ggaggaccga aggagctaac cgcttttttg cacaacatgg 1440
   gggatcatgt aactcgcctt gatcgttggg aaccggagct gaatgaagcc ataccaaacg 1500
   acgagcgtga caccacgatg cctgtagcaa tggcaacaac gttgcgcaaa ctattaactg 1560
   gcgaactact tactctagct tcccggcaac aattaataga ctggatggag gcggataaag 1620
   ttgcaggacc acttctgcgc tcggcccttc cggctggctg gtttattgct gataaatctg 1680
   gagccggtga gcgtgggtct cgcggtatca ttgcagcact ggggccagat ggtaagccct 1740
   cccgtatcgt agttatctac acgacgggga gtcaggcaac tatggatgaa cgaaatagac 1800
   agatcgctga gataggtgcc tcactgatta agcattggta actgtcagac caagtttact 1860
   catatatact ttagattgat ttaaaacttc atttttaatt taaaaggatc taggtgaaga 1920
   tcctttttga taatctcatg accaaaatcc cttaacgtga gttttcgttc cactgagcgt 1980
   cagaccccgt agaaaagatc aaaggatctt cttgagatcc tttttttctg cgcgtaatct 2040
   gctgcttgca aacaaaaaaa ccaccgctac cagcggtggt ttgtttgccg gatcaagagc 2100
   taccaactct ttttccgaag gtaactggct tcagcagagc gcagatacca aatactgtcc 2160
   ttctagtgta gccgtagtta ggccaccact tcaagaactc tgtagcaccg cctacatacc 2220
   tcgctctgct aatcctgtta ccagtggctg ctgccagtgg cgataagtcg tgtcttaccg 2280
   ggttggactc aagacgatag ttaccggata aggcgcagcg gtcgggctga acggggggtt 2340
   cgtgcacaca gcccagcttg gagcgaacga cctacaccga actgagatac ctacagcgtg 2400
   agctatgaga aagcgccacg cttcccgaag ggagaaaggc ggacaggtat ccggtaagcg 2460
   gcagggtcgg aacaggagag cgcacgaggg agcttccagg gggaaacgcc tggtatcttt   2520
   atagtcctgt cgggtttcgc cacctctgac ttgagcgtcg atttttgtga tgctcgtcag   2580
   gggggcggag cctatggaaa aacgccagca acgcggcctt tttacggttc ctggcctttt   2640
   gctggccttt tgctcacatg ttctttcctg cgttatcccc tgattctgtg gataaccgta   2700
   ttaccgcctt tgagtgagct gataccgctc gccgcagccg aacgaccgag cgcagcgagt   2760
   cagtgagcga ggaagcggaa gagcgcccaa tacgcaaacc gcctctcccc gcgcgttggc   2820
   cgattcatta atgcagctgg cacgacaggt ttcccgactg gaaagcgggc agtgagcgca   2880
   acgcaattaa tgtgagttag ctcactcatt aggcacccca ggctttacac tttatgcttc   2940
   cggctcgtat gttgtgtgga attgtgagcg gataacaatt tcacacagga aacagctatg   3000
   accatgatta cgccaagcgc gcaattaacc ctcactaaag ggaacaaaa              3049

## Description

The present invention relates to a method of modifying a target sequence in the genome of a eukaryotic cell as characterised in the appended claims, the method comprising the step: (a) introducing into the cell a fusion protein comprising a DNA-binding domain of a Tal effector protein and a non-specific cleavage domain of a restriction nuclease or a nucleic acid molecule encoding the fusion protein in expressible form, wherein the fusion protein specifically binds within the target sequence and introduces a double strand break within the target sequence. The present invention further relates to the method of the invention, wherein the modification of the target sequence is by homologous recombination with a donor nucleic acid sequence further comprising the step: (b) introducing a nucleic acid molecule into the cell, wherein the nucleic acid molecule comprises the donor nucleic acid sequence and regions homologous to the target sequence. The present invention also relates to a method of producing a non-human mammal or vertebrate carrying a modified target sequence in its genome.

With the complete elucidation of the human, mouse and other mammalian genome sequences a major challenge is the functional characterization of every gene within the genome and the identification of gene products and their molecular interaction network. In the past two decades the mouse has developed into the prime mammalian genetic model to study human biology and disease because methods are available that allow the production of targeted, predesigned mouse mutants. This reverse genetics approach that enables the production of germ line and conditional knockout mice by gene targeting, relies on the use of murine embryonic stem (ES) cell lines. ES cell lines exhibit unique properties such that they are able, once established from the inner cell mass of a mouse blastocyst, to renew indefinitely in cell culture while retaining their early pluripotent differentiation state. This property allows to grow ES cells in large numbers and, since most mutagenesis methods are inefficient, to select rare genetic variants that are expanded into a pure stem cell clone that harbours a specific genetic alteration in the target gene. Upon introduction of ES cells into mouse blastocysts and subsequent embryo transfer these cells contribute to all cell types of the developing chimaeric embryo, including the germ line. By mating of germ line chimaeras to normal mice a genetic modification engineered in ES cells is inherited to their offspring and thereby transferred into the mouse germ line.

The basis for reverse mouse genetics was initially established in the decade of 1980-90 in three steps and the basic scheme that is followed since that time is essentially unchanged. The first of these steps was the establishment of ES cell lines from cultured murine blastocysts and of culture conditions that maintain their pluripotent differentiation state *in vitro* (Evans MJ, Kaufman MH., Nature 1981; 292:154-6; Martin GR. Proc Natl Acad Sci U S A 1981; 78:7634-8). A few years later it was first reported that ES cells, upon microinjection into blastocysts, are able to colonize the germ line in chimaeric mice (Bradley A, Evans M, Kaufman MH, Robertson E., Nature 1984; 309:255-6; Gossler A, Doetschman T, Korn R, Serfling E, Kemler R., Proc Natl Acad Sci U S A 1986; 83:9065-9). The third step concerns the technology to introduce pre-planned, inactivating mutations into target genes in ES cells by homologous recombination between a gene targeting vector and endogenous loci (gene targeting). Gene targeting allows the introduction of pre-designed, site-specific modifications into the mouse genome (Capecchi MR. Trends Genet 1989; 5:70-6). Since the first demonstration of homologous recombination in ES cells in 1987 (Thomas KR, Capecchi MR., Cell 1987; 51:503-12) and the establishment of the first knockout mouse strain in 1989 (Schwartzberg PL, Goff SP, Robertson EJ., Science 1989; 246:799-803) gene targeting was adopted to many other genes and has been used in the last decades to generate more than 3000 knockout mouse strains that provided a wealth of information on in vivo gene functions (Collins FS, Rossant J, Wurst W., Cell 2007; 128:9-13; Capecchi, M. R., Nat Rev Genet 2005; 6: 507-12).

Targeted gene inactivation in ES cells can be achieved through the insertion of a selectable marker (mostly the neomycin phosphotransferase gene, neo) into an exon of the target gene or the replacement of one or more exons. The mutant allele is initially assembled in a specifically designed gene targeting vector such that the selectable marker is flanked at both sides with genomic segments of the target gene that serve as homology regions to initiate homologous recombination. The frequency of homologous recombination increases with the length of these homology arms. Usually arms with a combined length of 10-15 kb are cloned into standard, high copy plasmid vectors that accommodate up to 20 kb of foreign DNA. To select against random vector integrations a negative selectable marker, such as the *Herpes simplex* thymidine kinase or diphtheria toxin gene, can be included at one end of the targeting vector. Upon electroporation of such a vector into ES cells and the selection of stable integrants, clones that underwent a homologous recombination event can be identified through the analysis of genomic DNA using a PCR or Southern blot strategy. Using such standard gene targeting vectors the efficiency at which homologous recombinant ES cell clones are obtained is the range of 0.1% to 10% as compared to the number of stable transfected (Neo resistant) ES cell clones. This rate depends on the length of the vector homology region, the degree of sequence identity of this region with the genomic DNA of the ES cell line and likely on the differential accessibility of individual genomic loci to homologous recombination. Optimal rates are achieved with longer homology regions and by the use of genomic fragments that exhibit sequence identity to the genome of the ES cell line, i.e. both should be isogenic and derived from the same inbred mouse strain (te Riele H, Maandag ER, Berns A. 1992. Proc Natl Acad Sci U S A 89:5128-5132). Since the frequency of stable transfection of ES cells by electroporation is about 10⁻⁴ (i.e. 1 Neo resistant cone from 10.000 electroporated cells), the absolute efficiency of obtaining homologous recombinant ES cells falls in the range of 10⁻⁵ - 10⁻⁷ (Cheah SS, Behringer RR., Methods Mol Biol 2000; 136: 455-63; DeChiara TM.; Methods Mol Biol 2001; 158: 19-45; Hasty P, Abuin A, Bradley A., 2000, In Gene Targeting: a practical approach, ed. AL Joyner, pp. 1-35. Oxford: Oxford University Press; Nagy A, Gertsenstein M, Vintersten K, Behringer R., 2003. Manipulating the Mouse Embryo. Cold Spring Harbour, New York: Cold Spring Harbour Laboratory Press)

Upon the isolation of recombinant ES cell clones modified ES cells are injected into blastocysts to transmit the mutant allele through the germ line of chimaeras and to establish a mutant strain. Through interbreeding of heterozygous mutants homozygotes are obtained that can be used for phenotype analysis.

Using the "classical" gene targeting approach described above germ line mutants are obtained that harbour the knockout mutation in all cells throughout development. This strategy identifies the first essential function of a gene during ontogeny. If the gene product fulfils an important role in development its inactivation can lead to embryonic lethality precluding further analysis in adult mice. In general about 30% of all knockout mouse strains exhibit an embryonic lethal phenotype, for specific classes of genes, e.g. those regulating angiogenesis, this rate can reach 100%. To avoid embryonic lethality and to study gene function only in specific cell types Gu et al. (Gu H, Marth JD, Orban PC, Mossmann H, Rajewsky K., Science 1994; 265:103-6) introduced a modified, conditional gene targeting scheme that allows to restrict gene inactivation to specific cell types or developmental stages. In a conditional mutant, gene inactivation is achieved by the insertion of two 34 bp recognition (loxP) sites of the site-specific DNA recombinase Cre into introns of the target gene such that recombination results in the deletion of loxP-flanked exons. Conditional mutants initially require the generation of two mouse strains: one strain harbouring a loxP flanked gene segment obtained by gene targeting in ES cells and a second, transgenic strain expressing Cre recombinase in one or several cell types. The conditional mutant is generated by crossing these two strains such that target gene inactivation occurs in a spatial and temporal restricted manner, according to the pattern of recombinase expression in the Cre transgenic strain (Nagy A, Gertsenstein M, Vintersten K, Behringer R. 2003. Manipulating the Mouse Embryo, third edition ed. Cold Spring Harbour, New York: Cold Spring Harbour Laboratory Press; Torres RM, Kühn R. 1997. Laboratory protocols for conditional gene targeting. Oxford: Oxford University Press). Conditional mutants have been used to address various biological questions which could not be resolved with germ line mutants, often because a null allele results in an embryonic or neonatal lethal phenotype.

Taken together, gene targeting in ES cells has revolutionised the *in vivo* analysis of mammalian gene function using the mouse as genetic model system. However, since germ line competent ES cell lines that can be genetically modified could be established only from mice, this reverse genetics approach is presently restricted to this rodent species. The exception from this rule is achieved by homologous recombination in primary cells from pig and sheep followed by the transplantation of nuclei from recombined somatic cells into enucleated oocytes (cloning) (Lai L, Prather RS. 2003. Reprod Biol Endocrinol 2003; 1:82; Gong M, Rong YS. 2003. Curr Opin Genet Dev 13:215-220). Since this methodology is inefficient and time consuming it does not have the potential to develop into a simple routine procedure.

Although the generation of targeted mouse mutants via genome engineering in ES cells and the derivation of germ line transmitting chimaeras is established as a routine procedure this approach typically requires 1-2 years of hands on work for vector construction, ES cell culture and selection and the breeding of chimaeras. Typical problems that are encountered during a gene targeting project are the low efficiency of homologous recombination in ES cells and the loss of the germ line competence of ES cells during the long *in vitro* culture and selection phase. Therefore, the successful generation of even a single line of knockout mice requires considerable time, the combined efforts of specialists in molecular biology, ES cell culture and embryo manipulation, and the associated technical infrastructure.

Experiments in model systems have demonstrated that the frequency of homologous recombination of a gene targeting vector is strongly increased if a double-strand break is induced within its chromosomal target sequence. Using the yeast homing endonuclease I-SceI, that cuts DNA at an 18 base pair-long recognition site, it was initially shown that homologous recombination and gene targeting are stimulated over 1000-fold in mammalian cells when a recognition site is inserted into a target gene and I-SceI is expressed in these cells (Rouet, P., Smih, F., Jasin, M.; Mol Cell Biol 1994; 14: 8096-8106; Rouet, P., Smih, F. Jasin, M.; Proc Natl Acad Sci USA 1994; 91: 6064-6068). In the absence of a gene targeting vector for homology directed repair, the cells frequently close the double-strand break by non-homologous end-joining (NHEJ). Since this mechanism is error-prone it frequently leads to the deletion or insertion of multiple nucleotides at the cleavage site. If the cleavage site is located within the coding region of a gene it is thereby possible to identify and select mutants that exhibit reading frameshift mutations from a mutagenised population and that represent non-functional knockout alleles of the targeted gene.

In the past, zinc finger nucleases (ZFNs) were developed as a method to apply the stimulatory power of double strand breaks to sequences of endogenous genes, without the need to introduce an artificial nuclease recognition site. Using zinc finger nucleases in the absence of a gene targeting vector for homology directed repair, knockout alleles were generated in mammalian cell lines and knockout zebra fish and rats were obtained upon the expression of ZFN mRNA in one cell embryos (Santiago Y, Chan E, Liu PQ, Orlando S, Zhang L, Urnov FD, Holmes MC, Guschin D, Waite A, Miller JC, Rebar EJ, Gregory PD, Klug A, Collingwood TN.; Proc Natl Acad Sci U S A 2008; 105:5809-5814; Doyon Y, McCammon JM, Miller JC, Faraji F, Ngo C, Katibah GE, Amora R, Hocking TD, Zhang L, Rebar EJ, Gregory PD, Urnov FD, Amacher SL.; Nat Biotechnol 2008; 26:702-708; Geurts AM, Cost GJ, Freyvert Y, Zeitler B, Miller JC, Choi VM, Jenkins SS, Wood A, Cui X, Meng X, Vincent A, Lam S, Michalkiewicz M, Schilling R, Foeckler J, Kalloway S, Weiler H, Menoret S, Anegon I, Davis GD, Zhang L, Rebar EJ, Gregory PD, Urnov FD, Jacob HJ, Buelow R.; Science 2009; 325:433).

Furthermore, zinc finger nucleases were used in the presence of exogenous gene targeting vectors that contain homology regions to the target gene for homology driven repair of the double strand break through gene conversion. This methodology has been applied to gene engineering in mammalian cell lines and gene correction in primary human cells (Urnov FD, Miller JC, Lee YL, Beausejour CM, Rock JM, Augustus S, Jamieson AC, Porteus MH, Gregory PD, Holmes MC.; Nature 2005; 435:646-651; Porteus MH, Baltimore D. 2003. Science 300:763; Hockemeyer D, Soldner F, Beard C, Gao Q, Mitalipova M, DeKelver RC, Katibah GE, Amora R, Boydston EA, Zeitler B, Meng X, Miller JC, Zhang L, Rebar EJ, Gregory PD, Urnov FD, Jaenisch R.; Nat Biotechnol 2009; 27:851-857).

Although the use of zinc finger nucleases results in a higher frequency of homologous recombination, considerable efforts and time are required to design zinc finger proteins that bind a new DNA target sequence at high efficiency. In addition, it has been calculated that using the presently available resources only one zinc finger nuclease could be found within a target region of 1000 base pairs of the mammalian genome (Maeder, et al. 2008 Mol Cell 31(2): 294-301; Maeder, et al. 2009 Nat Protoc 4(10): 1471-501).

The technical problem underlying the present invention is thus the provision of improved means and methods for modifying the genome of eukaryotic cells, such as e.g. mammalian or vertebrate cells.

The solution to this technical problem is achieved by providing the embodiments characterised in the claims.

Accordingly, the present invention relates to a method of modifying a target sequence in the genome of a eukaryotic cell as characterised in the appended claims, the method comprising the step: (a) introducing into the cell a fusion protein comprising a DNA-binding domain of a Tal effector protein and a non-specific cleavage domain of a restriction nuclease or a nucleic acid molecule encoding the fusion protein in expressible form, wherein the fusion protein specifically binds within the target sequence and introduces a double strand break within the target sequence.

The term "modifying" as used in accordance with the present invention refers to site-specific genomic manipulations resulting in changes in the nucleotide sequence. The genetic material comprising these changes in its nucleotide sequence is also referred to herein as the "modified target sequence". The term "modifying" includes, but is not limited to, substitution, insertion and deletion of one or more nucleotides within the target sequence.

The term "substitution", as used herein, refers to the replacement of nucleotides with other nucleotides. The term includes for example the replacement of single nucleotides resulting in point mutations. Said point mutations can lead to an amino acid exchange in the resulting protein product but may also not be reflected on the amino acid level. Also encompassed by the term "substitution" are mutations resulting in the replacement of multiple nucleotides, such as for example parts of genes, such as parts of exons or introns as well as replacement of entire genes.

The term "insertion" in accordance with the present invention refers to the incorporation of one or more nucleotides into a nucleic acid molecule. Insertion of parts of genes, such as parts of exons or introns as well as insertion of entire genes is also encompassed by the term "insertion". When the number of inserted nucleotides is not dividable by three, the insertion can result in a frameshift mutation within a coding sequence of a gene. Such frameshift mutations will alter the amino acids encoded by a gene following the mutation. In some cases, such a mutation will cause the active translation of the gene to encounter a premature stop codon, resulting in an end to translation and the production of a truncated protein. When the number of inserted nucleotides is instead dividable by three, the resulting insertion is an "in-frame insertion". In this case, the reading frame remains intact after the insertion and translation will most likely run to completion if the inserted nucleotides do not code for a stop codon. However, because of the inserted nucleotides, the finished protein will contain, depending on the size of the insertion, one or multiple new amino acids that may effect the function of the protein.

The term "deletion" as used in accordance with the present invention refers to the loss of nucleotides or part of genes, such as exons or introns as well as entire genes. As defined with regard to the term "insertion", the deletion of a number of nucleotides that is not evenly dividable by three will lead to a frameshift mutation, causing all of the codons occurring after the deletion to be read incorrectly during translation, potentially producing a severely altered and most likely non-functional protein. If a deletion does not result in a frameshift mutation, i.e. because the number of nucleotides deleted is dividable by three, the resulting protein is nonetheless altered as the finished protein will lack, depending on the size of the deletion, several amino acids that may effect the function of the protein.

The above defined modifications are not restricted to coding regions in the genome, but can also occur in non-coding regions of the target genome, for example in regulatory regions such as promoter or enhancer elements or in introns.

Examples of modifications of the target genome include, without being limited, the introduction of mutations into a wild type gene in order to analyse its effect on gene function; the replacement of an entire gene with a mutated gene or, alternatively, if the target sequence comprises mutation(s), the alteration of these mutations to identify which mutation is causative of a particular effect; the removal of entire genes or proteins or the removal of regulatory elements from genes or proteins as well as the introduction of fusion-partners, such as for example purification tags such as the his-tag or the tap-tag etc.

In accordance with the present invention, the term "target sequence in the genome" refers to the genomic location that is to be modified by the method of the invention. The "target sequence in the genome" comprises but is not restricted to the nucleotide(s) subject to the particular modification. Furthermore, the term "target sequence in the genome" also comprises regions for binding of homologous sequences of a second nucleic acid molecule. In other words, the term "target sequence in the genome" also comprises the sequence surrounding the relevant nucleotide(s) to be modified. Preferably, the term "target sequence" refers to the entire gene to be modified.

The term "fusion protein comprising a DNA-binding domain of a Tal effector protein and a non-specific cleavage domain of a restriction nuclease", as used in accordance with the present invention, refers to a fusion protein comprising a DNA-binding domain, wherein the DNA-binding domain comprises or consists of Tal effector motifs and the non-specific cleavage domain of a restriction nuclease. The fusion protein employed in the method of the invention retains or essentially retains the enzymatic activity of the native (restriction) endonuclease. In accordance with the present invention, (restriction) endonuclease function is essentially retained if at least 60% of the biological activity of the endonuclease activity are retained. Preferably, at least 75% or at least 80% of the endonuclease activity are retained. More preferred is that at least 90% such as at least 95%, even more preferred at least 98% such as at least 99% of the biological activity of the endonuclease are retained. Most preferred is that the biological activity is fully, i.e. to 100%, retained. Also in accordance with the invention, fusion proteins having an increased biological activity compared to the endogenous endonuclease, i.e. more than 100% activity. Methods of assessing biological activity of (restriction) endonucleases are well known to the person skilled in the art and include, without being limiting, the incubation of an endonuclease with recombinant DNA and the analysis of the reaction products by gel electrophoresis (Bloch KD.; Curr Protoc Mol Biol 2001; Chapter 3:Unit 3.2).

The term "Tal effector protein", as used herein, refers to proteins belonging to the TAL (transcription activator-like) family of proteins. These proteins are expressed by bacterial plant pathogens of the genus *Xanthomonas.* Members of the large TAL effector family are key virulence factors of *Xanthomonas* and reprogram host cells by mimicking eukaryotic transcription factors. The pathogenicity of many bacteria depends on the injection of effector proteins via type III secretion into eukaryotic cells in order to manipulate cellular processes. TAL effector proteins from plant pathogenic *Xanthomonas* are important virulence factors that act as transcriptional activators in the plant cell nucleus. PthXo1, a TAL effector protein of a *Xanthomonas* rice pathogen, activates expression of the rice gene Os8N3, allowing *Xanthomonas* to colonize rice plants. TAL effector proteins are characterized by a central domain of tandem repeats, i.e. a DNA-binding domain as well as nuclear localization signals (NLSs) and an acidic transcriptional activation domain. Members of this effector family are highly conserved and differ mainly in the amino acid sequence of their repeats and in the number of repeats. The number and order of repeats in a TAL effector protein determine its specific activity. These repeats are referred to herein as "TAL effector motifs". One exemplary member of this effector family, AvrBs3 from *Xanthomonas campestris pv. vesicatoria,* contains 17.5 repeats and induces expression of UPA (up-regulated by AvrBs3) genes, including the Bs3 resistance gene in pepper plants (Kay, et al. 2005 Mol Plant Microbe Interact 18(8): 838-48; Kay, S. and U. Bonas 2009 Curr Opin Microbiol 12(1): 37-43). The repeats of AvrBs3 are essential for DNA binding of AvrBs3 and represent a distinct type of DNA binding domain. The mechanism of sequence specific DNA recognition has been elucidated by recent studies on the AvrBs3, Hax2, Hax3 and Hax4 proteins that revealed the TAL effectors' DNA recognition code (Boch, J., et al. 2009 Science 326: 1509-12).

Tal effector motifs or repeats are 32 to 34 amino acid protein sequence motifs. The amino acid sequences of the repeats are conserved, except for two adjacent highly variable residues (at positions 12 and 13) that determine specificity towards the DNA base A, G, C or T. In other words, binding to DNA is mediated by contacting a nucleotide of the DNA double helix with the variable residues at position 12 and 13 within the Tal effector motif of a particular Tal effector protein (Boch, J., et al. 2009 Science 326: 1509-12).Therefore, a one-to-one correspondence between sequential amino acid repeats in the Tal effector proteins and sequential nucleotides in the target DNA was found. Each Tal effector motif primarily recognizes a single nucleotide within the DNA substrate. For example, the combination of histidine at position 12 and aspartic acid at position 13 specifically binds cytidine; the combination of asparagine at both position 12 and position 13 specifically binds guanosine; the combination of asparagine at position 12 and isoleucine at position 13 specifically binds adenosine and the combination of asparagine at position 12 and glycine at position 13 specifically binds thymidine, as shown in Example 1 below. Binding to longer DNA sequences is achieved by linking several of these Tal effector motifs in tandem to form a "DNA-binding domain of a Tal effector protein". Thus, the term "DNA-binding domain of a Tal effector protein" relates to DNA-binding domains found in naturally occurring Tal effector proteins as well as to DNA-binding domains designed to bind to a specific target nucleotide sequence as described in the examples below. The use of such DNA-binding domains of Tal effector proteins for the creation of Tal effector motif - nuclease fusion proteins that recognize and cleave a specific target sequence depends on the reliable creation of DNA-binding domains of Tal effector proteins that can specifically recognize said particular target. Methods for the generation of DNA-binding domains of Tal effector proteins are disclosed in the appended examples of this application.

Preferably, the DNA-binding domain is derived from the Tal effector motifs found in naturally occurring Tal effector proteins, such as for example Tal effector proteins selected from the group consisting of AvrBs3, Hax2, Hax3 or Hax4 (Bonas et al. 1989. Mol Gen Genet 218(1): 127-36; Kay et al. 2005 Mol Plant Microbe Interact 18(8): 838-48).

Preferably, the restriction nuclease is an endonuclease. The terms "endonuclease" and "restriction endonuclease" are used herein according to the well-known definitions provided by the art. Both terms thus refer to enzymes capable of cutting nucleic acids by cleaving the phosphodiester bond within a polynucleotide chain. Preferably, the endonuclease is a type II S restriction endonuclease, such as for example Fokl, AlwI, SfaNI, Sapl, PleI, NmeAIII, Mboll, Mlyl, Mmel, HpYAV, Hphl, Hgal, Faul, Earl, Ecil, BtgZI, CspCI, BspQI, BspMI, BsaXI, Bsgl, Bsel, BpuEIBmrIBcgIBbvI, Bael, BbsIAlwI, or Acul or a type III restriction endonuclease (e.g. EcoP1I, EcoP15I, Hinflll). Also envisaged herein are meganucleases, such as for example I-SceI. More preferably, the endonuclease is Fokl endonuclease. FokI is a bacterial type IIS restriction endonuclease. It recognises the non-palindromic penta-deoxyribonucleotide 5'-GGATG-3': 5'-CATCC-3' in duplex DNA and cleaves 9/13 nucleotides downstream of the recognition site. Fokl does not recognise any specific-sequence at the site of cleavage. Once the DNA-binding domain (either of the naturally occurring endonuclease, e.g. Fokl or, in accordance with the present invention, of the fusion protein comprising a DNA-binding domain of a Tal effector protein and a nuclease domain) is anchored at the recognition site, a signal is transmitted to the endonuclease domain and cleavage occurs. The distance of the cleavage site to the DNA-binding site of the fusion protein depends on the particular endonuclease present in the fusion protein. For example, the fusion protein employed in the examples of the present invention cleaves in the middle of a 6 bp sequence that is flanked by the two binding sites of the fusion protein. As a further example, naturally occurring endonucleases such as Fokl and EcoP15I cut at 9/13 and 27 bp distance from the DNA binding site, respectively.

Envisaged in accordance with the present invention are fusion proteins that are provided as functional monomers comprising a DNA-binding domain of a Tal effector protein coupled with a single nuclease domain. The DNA-binding domain of a Tal effector protein and the cleavage domain of the nuclease may be directly fused to one another or may be fused via a linker.

The term "linker" as used in accordance with the present invention relates to a sequel of amino acids (i.e. peptide linkers) as well as to non-peptide linkers.

Peptide linkers as envisaged by the present invention are (poly)peptide linkers of at least 1 amino acid in length. Preferably, the linkers are 1 to 100 amino acids in length. More preferably, the linkers are 5 to 50 amino acids in length and even more preferably, the linkers are 10 to 20 amino acids in length. It is well known to the skilled person that the nature, i.e. the length and/or amino acid sequence of the linker may modify or enhance the stability and/or solubility of the molecule. Thus, the length and sequence of a linker depends on the composition of the respective portions of the fusion protein of the invention.

The skilled person is aware of methods to test the suitability of different linkers. For example, the properties of the molecule can easily be tested by testing the nuclease activity as well as the DNA-binding specificity of the respective portions of the fusion protein of the invention.

It will be appreciated by the skilled person that when the fusion protein of the invention is provided as a nucleic acid molecule encoding the fusion protein in expressible form, the linker is a peptide linker also encoded by said nucleic acid molecule.
The term "non-peptide linker", as used in accordance with the present invention, refers to linkage groups having two or more reactive groups but excluding peptide linkers as defined above. For example, the non-peptide linker may be a polymer having reactive groups at both ends, which individually bind to reactive groups of the individual portions of the fusion protein of the invention, for example, an amino terminus, a lysine residue, a histidine residue or a cysteine residue. The reactive groups of the polymer include an aldehyde group, a propionic aldehyde group, a butyl aldehyde group, a maleimide group, a ketone group, a vinyl sulfone group, a thiol group, a hydrazide group, a carbonyldimidazole (CDI) group, a nitrophenyl carbonate (NPC) group, a trysylate group, an isocyanate group, and succinimide derivatives. Examples of succinimide derivatives include succinimidyl propionate (SPA), succinimidyl butanoic acid (SBA), succinimidyl carboxymethylate (SCM), succinimidyl succinamide (SSA), succinimidyl succinate (SS), succinimidyl carbonate, and N-hydroxy succinimide (NHS). The reactive groups at both ends of the non-peptide polymer may be the same or different. For example, the non-peptide polymer may have a maleimide group at one end and an aldehyde group at another end.

In a preferred embodiment, the linker is a peptide linker.

More preferably, the peptide linker consists of seven glycine residues.

Without wishing to be bound by theory, the present inventors believe that the mechanism of double-strand cleavage by a fusion protein of the invention requires dimerisation of the nuclease domain in order to cut the DNA substrate. Thus, in a preferred embodiment, at least two fusion proteins are introduced into the cell in step (a). Dimerisation of the fusion protein can result in the formation of homodimers if only one type of fusion protein is present or in the formation of heterodimers, when different types of fusion proteins are present. It is preferred in accordance with the present invention that at least two different types of fusion proteins having differing DNA-binding domains of a Tal effector protein are introduced into the cell. The at least two different types of fusion proteins can be introduced into the cell either separately or together. Also envisaged herein is a fusion protein, which is provided as a functional dimer via linkage of two subunits of identical or different fusion proteins prior to introduction into the cell. Suitable linkers have been defined above.

The term "nucleic acid molecule encoding the fusion protein in expressible form" refers to a nucleic acid molecule which, upon expression in a cell or a cell-free system, results in a functional fusion protein. Nucleic acid molecules as well as nucleic acid sequences, as used throughout the present description, include DNA, such as cDNA or genomic DNA, and RNA. Preferably, embodiments reciting "RNA" are directed to mRNA. Furthermore included is genomic RNA, such as in case of RNA of RNA viruses.

It will be readily appreciated by the skilled person that more than one nucleic acid molecule may encode a fusion protein in accordance with the present invention due to the degeneracy of the genetic code. Degeneracy results because a triplet code designates 20 amino acids and a stop codon. Because four bases exist which are utilized to encode genetic information, triplet codons are required to produce at least 21 different codes. The possible 4³ possibilities for bases in triplets give 64 possible codons, meaning that some degeneracy must exist. As a result, some amino acids are encoded by more than one triplet, i.e. by up to six. The degeneracy mostly arises from alterations in the third position in a triplet. This means that nucleic acid molecules having different sequences, but still encoding the same fusion protein can be employed in accordance with the present invention.

In accordance with the present invention, the term "specifically binds within the target sequence and introduces a double strand break within the target sequence" means that the fusion protein is designed such that statistically it only binds to a particular sequence and does not bind to an unrelated sequence elsewhere in the genome. Preferably, the fusion protein in accordance with the present invention comprises at least 18 Tal effector motifs. In other words, the DNA-binding domain of a Tal effector protein within said fusion protein is comprised of at least 18 Tal effector motifs. In the case of fusion proteins consisting of dimers as described above this means that each fusion protein monomer comprises at least nine Tal effector motifs. More preferably, each fusion protein comprises at least 12 Tal effector motifs, such as for example at least 14 or at least 16 Tal effector motifs. Methods for testing the DNA-binding specificity of a fusion protein in accordance with the present invention are known to the skilled person and include, without being limiting, transcriptional reporter gene assays and electrophoretic mobility shift assays (EMSA).

Preferably, the binding site of the fusion protein is up to 500 nucleotides, such as up to 250 nucleotides, up to 100 nucleotides, up to 50 nucleotides, up to 25 nucleotides, up to 10 nucleotides such as up to 5 nucleotides upstream (i.e. 5') or downstream (i.e. 3') of the nucleotide(s) that is/are modified in accordance with the present invention.

In a preferred embodiment of the present invention, the modification of the target sequence is by homologous recombination with a donor nucleic acid sequence further comprising the step: (b) introducing a nucleic acid molecule into the cell, wherein the nucleic acid molecule comprises the donor nucleic acid sequence and regions homologous to the target sequence.

The term "homologous recombination", is used according to the definitions provided in the art. Thus, it refers to a mechanism of genetic recombination in which two DNA strands comprising similar nucleotide sequences exchange genetic material. Cells use homologous recombination during meiosis, where it serves to rearrange DNA to create an entirely unique set of haploid chromosomes, but also for the repair of damaged DNA, in particular for the repair of double strand breaks. The mechanism of homologous recombination is well known to the skilled person and has been described, for example by Paques and Haber ( Paques F, Haber JE.; Microbiol Mol Biol Rev 1999; 63:349-404)

In accordance with the present invention, the term "donor nucleic acid sequence" refers to a nucleic acid sequence that serves as a template in the process of homologous recombination and that carries the modification that is to be introduced into the target sequence. By using this donor nucleic acid sequence as a template, the genetic information, including the modifications, is copied into the target sequence within the genome of the cell. In non-limiting examples, the donor nucleic acid sequence can be essentially identical to the part of the target sequence to be replaced, with the exception of one nucleotide which differs and results in the introduction of a point mutation upon homologous recombination or it can consist of an additional gene previously not present in the target sequence.

In accordance with the method of modifying a target sequence of the present invention, the nucleic acid molecule introduced into the cell in step (b) comprises the donor nucleic acid sequence as defined above as well as additional regions that are homologous to the target sequence. It will be appreciated by one of skill in the art that the nucleic acid molecule to be introduced into the cell in step (b) may comprise both the nucleic acid molecule encoding the fusion protein and the nucleic acid molecule comprising the donor nucleic acid sequence and regions homologous to the target sequence. Alternatively, the nucleic acid molecule of step (b) may be a further nucleic acid molecule, to be introduced in addition to the nucleic acid molecule encoding the fusion protein in accordance with step (a).

The term "regions homologous to the target sequence" (also referred to as "homology arms" herein), in accordance with the present invention, refers to regions having sufficient sequence identity to ensure specific binding to the target sequence. Methods to evaluate the identity level between two nucleic acid sequences are well known in the art. For example, the sequences can be aligned electronically using suitable computer programs known in the art. Such programs comprise BLAST (Altschul et al. (1990) J. Mol. Biol. 215, 403), variants thereof such as WU-BLAST (Altschul and Gish (1996) Methods Enzymol. 266, 460), FASTA (Pearson and Lipman (1988) Proc. Natl. Acad. Sci. USA 85, 2444) or implementations of the Smith-Waterman algorithm (SSEARCH, Smith and Waterman (1981) J. Mol. Biol., 147, 195). These programs, in addition to providing a pairwise sequence alignment, also report the sequence identity level (usually in percent identity) and the probability for the occurrence of the alignment by chance (P-value).

Preferably, the "regions homologous to the target sequence" have a sequence identity with the corresponding part of the target sequence of at least 95%, more preferred at least 97%, more preferred at least 98%, more preferred at least 99%, even more preferred at least 99.9% and most preferred 100%. The above defined sequence identities are defined only with respect to those parts of the target sequence which serve as binding sites for the homology arms. Thus, the overall sequence identity between the entire target sequence and the homologous regions of the nucleic acid molecule of step (b) of the method of modifying a target sequence of the present invention can differ from the above defined sequence identities, due to the presence of the part of the target sequence which is to be replaced by the donor nucleic acid sequence.

It is preferred that at least two regions homologous to the target sequence are present in the nucleic acid molecule of (b).
In accordance with the method of the present invention, step (a) of introducing the fusion protein into the cell and step (b) of introducing the nucleic acid molecule into the cell are either carried out concomitantly, i.e. at the same time or are carried out separately, i.e. individually and at different time points. When the steps are carried out concomitantly, both the fusion protein and the nucleic acid molecule can be administered in parallel, for example using two separate injection needles or can be mixed together and, for example, be injected using one needle.

In accordance with the present invention it was surprisingly found that it is possible to introduce gene modifications, including targeted gene modifications, into the genome of eukaryotic cells and to achieve an unexpectedly high frequency of homologous recombination of up to 10% by employing a fusion protein comprising a DNA-binding domain of a Tal effector protein and a non-specific cleavage domain of a restriction nuclease.

Performing the cleavage step of the method of the invention will frequently lead to spontaneous genome modifications through nucleotide loss associated with the repair of double strand breaks by nonhomologous end joining (NHEJ) repair. In addition, by providing a nucleic acid molecule comprising a donor nucleic acid sequence and regions homologous to the target sequence, targeted modification of a genome can be achieved with high specificity.

Several methods are known in the art for achieving an improved frequency of genetic modification. Such methods include, for example, the use of zinc finger nucleases for achieving homologous recombination. However, in order to design zinc finger proteins that bind a new DNA target sequence at high efficiency, considerable efforts and time are required. Furthermore, neighbouring zinc fingers generally influence each other. Thus, they cannot be simply combined into a larger protein in a combinatorial way in order to enhance sequence specificity. As a consequence, the addition of new zinc fingers to a preselected zinc finger protein requires a laborious screening and selection procedure for each individual step. Furthermore, due to the incompletely known DNA binding code and the limited resources of coding zinc finger domains, it is presently difficult to design a nuclease fused to a zinc finger protein specific to any given DNA target sequence. It has been calculated that using the presently available resources only one zinc finger nuclease could be found within a target region of 1000 basepairs of the mammalian genome (Maeder, et al. 2008 Mol Cell 31(2): 294-301; Maeder, et al. 2009 Nat Protoc 4(10): 1471-501).

Another method employed to achieve a target sequence specific DNA double strand break is the use of yeast derived meganucleases, representing restriction enzymes like I-SceI that binds to specific 18 bp recognition sequence that does not occur naturally in mammalian genomes. However, a combinatorial code for the DNA binding specificity of meganucleases has not been revealed. The redesign of the DNA binding domain of meganucleases allowed so far only the substitution of one or a few nucleotides within their natural binding sequence (Pâques and Duchateau, 2007 Curr Gene Ther 7(1): 49-66). Therefore, the choice of mega nuclease target sites is very limited and it is presently not possible to design new meganucleases that bind to any preferred target region within mammalian genomes.

In contrast to these methods, the Tal effector DNA binding domains provide a simple combinatorial code for the construction of new DNA binding proteins with chosen specificity that can be applied to any target sequence within any genome.

In accordance with the present invention a method of introducing genetic modifications into a target genome is provided that overcomes the above discussed problems currently faced by the skilled person. In particular, any number of nucleotide-specific Tal effector motifs can be combined to form a sequence-specific DNA-binding domain to be employed in the fusion protein in accordance with the present invention. Thus, any sequence of interest can now be targeted in a cost-effective, easy and fast way.

In a preferred embodiment, the cells are analysed for successful modification of the target genome.
Methods for analysing for the presence or absence of a modification are well known in the art and include, without being limiting, assays based on physical separation of nucleic acid molecules, sequencing assays as well as cleavage and digestion assays and DNA analysis by the polymerase chain reaction (PCR).

Examples for assays based on physical separation of nucleic acid molecules include without limitation MALDI-TOF, denaturating gradient gel electrophoresis and other such methods known in the art, see for example Petersen et al., Hum. Mutat. 20 (2002) 253-259; Hsia et al., Theor. Appl. Genet. 111 (2005) 218-225; Tost and Gut, Clin. Biochem. 35 (2005) 335-350; Palais et al., Anal. Biochem. 346 (2005) 167-175.

Examples for sequencing assays comprise without limitation approaches of sequence analysis by direct sequencing, fluorescent SSCP in an automated DNA sequencer and Pyrosequencing. These procedures are common in the art, see e.g. Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

Examples for cleavage and digestion assays include without limitation restriction digestion assays such as restriction fragments length polymorphism assays (RFLP assays), RNase protection assays, assays based on chemical cleavage methods and enzyme mismatch cleavage assays, see e.g. Youil et al., Proc. Natl. Acad. Sci. U.S.A. 92 (1995) 87-91; Todd et al., J. Oral Maxil. Surg. 59 (2001) 660-667; Amar et al., J. Clin. Microbiol. 40 (2002) 446-452.

Alternatively, instead of analysing the cells for the presence or absence of the desired modification, successfully modified cells may be selected by incorporation of appropriate selection markers. Selection markers include positive and negative selection markers, which are well known in the art and routinely employed by the skilled person. Non-limiting examples of selection markers include dhfr, gpt, neomycin, hygromycin, dihydrofolate reductase, G418 or glutamine synthase (GS) (Murphy et al., Biochem J. 1991, 227:277; Bebbington et al., Bio/Technology 1992, 10:169). Using these markers, the cells are grown in selective medium and the cells with the highest resistance are selected. Also envisaged are combined positive-negative selection markers, which may be incorporated into the target genome by homologous recombination or random integration. After positive selection, the first cassette comprising the positive selection marker flanked by recombinase recognition sites is exchanged by recombinase mediated cassette exchange against a second, marker-less cassette. Clones containing the desired exchange cassette are then obtained by negative selection.

In a preferred embodiment of the method of the invention, the cell is selected from the group consisting of a mammalian or vertebrate cell.
In accordance with the present invention, the cell is an oocyte as defined in the claims.

As used herein the term "oocyte" refers to the female germ cell involved in reproduction, i.e. the ovum or egg cell. In accordance with the present invention, the term "oocyte" comprises both oocytes before fertilisation as well as fertilised oocytes, which are also called zygotes. Thus, the oocyte before fertilisation comprises only maternal chromosomes, whereas an oocyte after fertilisation comprises both maternal and paternal chromosomes. After fertilisation, the oocyte remains in a double-haploid status for several hours, in mice for example for up to 18 hours after fertilisation.

In a preferred embodiment of the method of the invention, the oocyte is a fertilised oocyte.

The term "fertilised oocyte", as used herein, refers to an oocyte after fusion with the fertilizing sperm. For a period of many hours (such as up to 18 hours in mice) after fertilisation, the oocyte is in a double-haploid state, comprising one maternal haploid pronucleus and one paternal haploid pronucleus. After migration of the two pronuclei together, their membranes break down, and the two genomes condense into chromosomes, thereby reconstituting a diploid organism. Preferably, the mammalian or avian oocyte used in the method of the present invention is a fertilised mammalian or avian oocyte in the double-haploid state.

The re-modelling of a fertilised oocyte into a totipotent zygote refers to one of the most complex cell transformations in biology. Remarkably, this transition occurs in the absence of transcription factors and therefore depends on mRNAs accumulated in the oocyte during oogenesis. A growing mouse oocyte, arrested at diplotene of its first meiotic prophase, transcribes and translates many of its own genes, thereby producing a store of proteins sufficient to support development up to the 8-cell stage. These transcripts guide oocytes on the two steps of oocyte maturation and egg activation to become zygotes. Typically, oocytes are ovulated and become competent for fertilisation before reaching a second arrest point. When an oocyte matures into an egg, it arrests in metaphase of its second meiotic division where transcription stops and translation of mRNA is reduced. At this point an ovulated mouse egg has a diameter of 0.085 mm, with a volume of ~ 300 picoliter it exceeds 1000-fold the size of a typical somatic cell (Nagy A, Gertsenstein M, Vintersten K, Behringer R., 2003. Manipulating the Mouse Embryo. Cold Spring Harbour, New York: Cold Spring Harbour Laboratory Press).

Life and the embryonic development of a mammal begin when sperm fertilises an egg to form a zygote. Fertilization of the egg triggers egg activation to complete the transformation to a zygote by signaling the completion of meiosis and the formation of pronuclei. At this stage the zygote represents a 1-cell embryo that contains a haploid paternal pronucleus derived from the sperm and a haploid maternal pronucleus derived from the oocyte. In mice this totipotent single cell stage lasts for only - 18 hours until the first mitotic division occurs.

As totipotent single entities, mammalian zygotes could be regarded as a preferred substrate for genome engineering since the germ line of the entire animal is accessible within a single cell. However, the experimental accessibility and manipulation of zygotes is severely restricted by the very limited numbers at which they are available (dozens-hundred) and their very short lasting nature. These parameters readily explain that the vast majority of genome manipulations, that occur at frequencies of below 10⁻⁵ like gene targeting, can be successfully performed only in cultured embryonic stem cells that are grown up to a number of 10⁷ cells in a single standard culture plate. The only exception from this rule concerns the generation of transgenic mice by pronuclear DNA injection that has been developed into a routine procedure due to the high frequency of transgene integration in up to 30% of injected zygotes (Palmiter RD, Brinster RL.; Annu Rev Genet 1986; 20:465-499). Since microinjected transgenes randomly integrate into the genome, this method can only be used to express additional genes on the background of an otherwise normal genome, but does not allow the targeted modification of endogenous genes.

An early report to characterise the potential of zygotes for targeted gene manipulation by Brinster (Brinster RL, Braun RE, Lo D, Avarbock MR, Oram F, Palmiter RD.; Proc Natl Acad Sci U S A 1989; 86:7087-7091), showed that this approach is not practical as only one targeted mouse was obtained from > 10.000 zygotes within 14 months of injections. Thus, Brinster *et al.* discouraged any further attempts in this direction. In addition to a low recombination frequency, Brinster *et al.* noted a high number of spontaneously occurring, undesired mutations within the targeted allele that severely compromised the function of the (repaired) histocompatibility class II gene. From the experience of Brinster *et al.* it could be extrapolated that the physiological, biochemical and epigenetic context of genomic DNA in the zygotic pronuclei are unfavourable to achieve targeted genetic manipulations, except for the random integration of transgenes that occurs at high frequency.

In addition, the biology of oocyte development into an embryo provides further obstacles for targeted genetic manipulations. In fertilized mammalian eggs, the two pronuclei that undergo DNA replication, do not fuse directly but approach each other and remain distinct until the membrane of each pronucleus has broken down in preparation for the zygote's first mitotic division that produces a 2-cell embryo. The 1-cell zygote stage is characterised by unique transcriptional and translation control mechanisms. One of the most striking features is a time-dependent mechanism, referred to as the zygotic clock, that delays the expression of the zygotic genome for ~24 h after fertilization, regardless of whether or not the one-cell embryo has completed S phase and formed a two-cell embryo (Nothias JY, Majumder S, Kaneko KJ, DePamphilis ML.; J Biol Chem 1995;270:22077-22080). In nature, the zygotic clock provides the advantage of delaying zygotic gene activation (ZGA) until chromatin can be remodelled from a condensed meiotic state to one in which selected genes can be transcribed. Since the paternal genome is completely packaged with protamines that must be replaced with histones, some genes might be prematurely expressed if ZGA were not prevented. Cell-specific transcription requires that newly minted zygotic chromosomes repress most, if not all, promoters until development progresses to a stage where specific promoters can be activated by specific enhancers or trans-activators. In the mouse, formation of a 2-cell embryo marks the transition from maternal gene dependence to zygotic gene activation (ZGA). Among mammals, the extent of development prior to zygotic gene activation (ZGA) varies among species from one to four cleavage events. Maternal mRNA degradation is triggered by meiotic maturation and 90% completed in 2-cell embryos, although maternal protein synthesis continues into the 8-cell stage. In addition to transcriptional control, the zygotic clock delays the translation of nascent mRNA until the 2-cell stage (Nothias JY, Miranda M, DePamphilis ML.; EMBO J 1996; 15:5715-5725). Therefore, the production of proteins from transgenic expression vectors injected into pronuclei is not achieved until 10 - 12 hours after the appearance of mRNA.

Geurts *et al.* have recently found that zinc finger nucleases can be used to induce double strand breaks in the genome of rat zygotes (Geurts AM, Cost GJ, Freyvert Y, Zeitler B, Miller JC, Choi VM, Jenkins SS, Wood A, Cui X, Meng X, Vincent A, Lam S, Michalkiewicz M, Schilling R, Foeckler J, Kalloway S, Weiler H, Menoret S, Anegon I, Davis GD, Zhang L, Rebar EJ, Gregory PD, Urnov FD, Jacob HJ, Buelow R.; Science 2009; 325:433). In this work the induced strand breaks were left for the endogenous, error prone DNA repair mechanism in order to later identify randomly occurring mutant alleles that lost or acquired nucleotides at the site of DNA cleavage. Provided that the zinc finger nuclease cleavage site is located within an exon region of a gene, a reading frame shift will occur in some of the mutant alleles and thereby lead to the production of truncated, non-functional protein. However, this method only leads to the generation of undirected mutations within the coding region of a gene. So far, it has not been possible to induce directed modifications like pre-planned nucleotide substitutions, to insert exogenous DNA sequences like reporter genes and recombinase recognition sites or to replace e.g. murine versus human coding regions.

The introduction of such genetic modifications requires homologous recombination of a specifically designed gene targeting vector with a target gene. Since procedures to achieve high rate homologous recombination in zygotes were not known so far, gene targeting in somatic cells and the subsequent nuclear transfer into enucleated oocytes from sheep and pig have been used as a surrogate technique (Lai L, Prather RS. 2003. Reprod Biol Endocrinol 2003; 1:82; Gong M, Rong YS. 2003. Curr Opin Genet Dev 13:215-220). However, both techniques are demanding and not very efficient and their combined use is impractical and not well suited for routine application.

In accordance with the present invention a method of introducing genetic modifications into a target genome is provided that overcomes the above discussed problems currently faced by the skilled person. Using the method of the present invention it is now possible to generate genetically modified animals faster, easier and more cost-effective than using any of the prior art methods.

In another preferred embodiment of the method of the invention, the fusion protein or the nucleic acid molecule encoding the fusion protein is introduced into the oocyte by microinjection.

Microinjection into the oocyte can be carried out by injection into the nucleus (before fertilisation), the pronucleus (after fertilisation) and/or by injection into the cytoplasm (both before and after fertilisation). When a fertilised oocyte is employed, injection into the pronucleus is carried out either for one pronucleus or for both pronuclei. Injection of the Tal-finger nuclease or of a DNA encoding the Tal-finger nuclease of step (a) of the method of modifying a target sequence of the present invention is preferably into the nucleus/pronucleus, while injection of an mRNA encoding the Tal-finger nuclease of step (a) is preferably into the cytoplasm. Injection of the nucleic acid molecule of step (b) is preferably into the nucleus/pronucleus. However, injection of the nucleic acid molecule of step (b) can also be carried out into the cytoplasm when said nucleic acid molecule is provided as a nucleic acid sequence having a nuclear localisation signal to ensure delivery into the nucleus/pronucleus. Preferably, the microinjection is carried out by injection into both the nucleus/pronucleus and the cytoplasm. For example, the needle can be introduced into the nucleus/pronucleus and a first amount of the Tal-finger nuclease and/or nucleic acid molecule are injected into the nucleus/pronucleus. While removing the needle from the oocyte, a second amount of the Tal-finger nuclease and/or nucleic acid molecule is injected into the cytoplasm.

Methods for carrying out microinjection are well known in the art and are described for example in Nagy et al. (Nagy A, Gertsenstein M, Vintersten K, Behringer R., 2003. Manipulating the Mouse Embryo. Cold Spring Harbour, New York: Cold Spring Harbour Laboratory Press) as well as in the examples herein below.

In another preferred embodiment of the method of the invention, the nucleic acid molecule of step (b) is introduced into the cell by microinjection.

In a more preferred embodiment, the nucleic acid molecule encoding the fusion protein in expressible form is mRNA.

In another preferred embodiment of the method of the invention, the regions homologous to the target sequence are localised at the 5' and 3' end of the donor nucleic acid sequence.

In this preferred embodiment, the donor nucleic acid sequence is flanked by the two regions homologous to the target sequence such that the nucleic acid molecule used in the method of the present invention consists of a first region homologous to the target sequence, followed by the donor nucleic acid sequence and then a second region homologous to the target sequence.

In a further preferred embodiment of the method of the invention, the regions homologous to the target sequence comprised in the nucleic acid molecule have a length of at least 400 bp each. More preferably, the regions each have a length of at least 500 nucleotides, such as at least 600 nucleotides, at least 750 bp nucleotides, more preferably at least 1000 nucleotides, such as at least 1500 nucleotides, even more preferably at least 2000 nucleotides and most preferably at least 2500 nucleotides. The maximum length of the regions homologous to the target sequence comprised in the nucleic acid molecule depends on the type of cloning vector used and can be up to a length 20.000 nucleotides each in E. coli high copy plasmids using the col EI replication origin (e.g. pBluescript) or up to a length of 300.000 nucleotides each in plasmids using the F-factor origin (e.g. in BAC vectors such as for example pTARBAC1).

In a further preferred embodiment of the method of the invention, the modification of the target sequence is selected from the group consisting of substitution, insertion and deletion of at least one nucleotide of the target sequence. Preferred in accordance with the present invention are substitutions, for example substitutions of 1 to 3 nucleotides and insertions of exogenous sequences, such as IoxP sites (34 nucleotides long) or cDNAs, such as for example for reporter genes. Such cDNAs for reporter genes can, for example, be up to 6 kb long.

In another preferred embodiment of the method of the invention, the cell is from a mammal selected from the group consisting of rodents, dogs, felides, monkeys, rabbits, pigs, or cows or the cell is from an avian selected from the group consisting of chickens, turkeys, pheasants, ducks, geese, quails and ratites including ostriches, emus and cassowaries or the cell is from a fish such a for example zebrafish, salmon, trout, common carp or coi carp.

All of the mammals, avians and fish described herein are well known to the skilled person and are taxonomically defined in accordance with the prior art and the common general knowledge of the skilled person.

Non-limiting examples of "rodents" are mice, rats, squirrels, chipmunks, gophers, porcupines, beavers, hamsters, gerbils, guinea pigs, degus, chinchillas, prairie dogs, and groundhogs.

Non-limiting examples of "dogs" include members of the subspecies *canis lupus familiaris* as well as wolves, foxes, jackals, and coyotes.

Non-limiting examples of "felides" include members of the two subfamilies: the pantherinae, including lions, tigers, jaguars and leopards and the felinae, including cougars, cheetahs, servals, lynxes, caracals, ocelots and domestic cats.

The term "primates", as used herein, refers to all monkey including for example cercopithecoid (old world monkey) or platyrrhine (new world monkey) as well as lemurs, tarsiers, apes and marmosets (Callithrix jacchus).

In one embodiment, the mammalian oocyte is not a human oocyte. In another embodiment, the fertilized oocyte is not a human oocyte.

The present invention further relates to a method of producing a non-human vertebrate or mammal carrying a modified target sequence in its genome as characterised in the appended claims.

In accordance with the present invention, the term "transferring a cell produced by the method of the invention into a pseudopregnant female host" includes the transfer of a fertilised oocyte but also the transfer of pre-implantation embryos of for example the 2-cell, 4-cell, 8-cell, 16-cell and blastocyst (70-to 100-cell) stage. Said pre-implantation embryos can be obtained by culturing the cell under appropriate conditions for it to develop into a pre-implantation embryo. Furthermore, injection or fusion of the cell with a blastocyst are appropriate methods of obtaining a pre-implantation embryo. Where the cell produced by the method of the invention is a somatic cell, derivation of induced pluripotent stem cells is required prior to transferring the cell into a female host such as for example prior to culturing the cell or injection or fusion of the cell with a pre-implantation embryo. Methods for transferring an oocyte or pre-implantation embryo to a pseudo pregnant female host are well known in the art and are, for example, described in Nagy et al., (Nagy A, Gertsenstein M, Vintersten K, Behringer R., 2003. Manipulating the Mouse Embryo. Cold Spring Harbour, New York: Cold Spring Harbour Laboratory Press).

It is further envisaged in accordance with the method of producing a non-human vertebrate or mammal carrying a modified target sequence in its genome that a step of analysis of successful genomic modification is carried out before transplantation into the female host. As a non-limiting example, the oocyte can be cultured to the 2-cell, 4-cell or 8-cell stage and one cell can be removed without destroying or altering the resulting embryo. Analysis for the genomic constitution, e.g. the presence or absence of the genomic modification, can then be carried out using for example PCR or southern blotting techniques or any of the methods described herein above. Such methods of analysis of successful genotyping prior to transplantation are known in the art and are described, for example in Peippo et al. (Peippo J, Viitala S, Virta J, Raty M, Tammiranta N, Lamminen T, Aro J, Myllymaki H, Vilkki J.; Mol Reprod Dev 2007; 74:1373-1378).

For this method of producing a non-human vertebrate or mammal, fertilisation of the oocyte is required. Said fertilisation can occur before the modification of the target sequence in step (a) in accordance with the method of producing a non-human vertebrate or mammal of the invention, i.e. a fertilised oocyte can be used for the method of modifying a target sequence in accordance with the invention. The fertilisation can also be carried out after the modification of the target sequence in step (a), i.e. a non-fertilised oocyte can be used for the method of modifying a target sequence in accordance with the invention, wherein the oocyte is subsequently fertilised before transfer into the pseudopregnant female host.

The step of analysing for the presence of the modification in the offspring delivered by the female host provides the necessary information whether or not the produced non-human vertebrate or mammal carries the modified target sequence in its genome. Thus, the presence of the modification is indicative of said offspring carrying a modified target sequence in its genome whereas the absence of the modification is indicative of said offspring not carrying the modified target sequence in its genome. Methods for analysing for the presence or absence of a modification have been detailed above.

The non-human vertebrate or mammal produced by the method of the invention is, inter alia, useful to study the function of genes of interest and the phenotypic expression/outcome of modifications of the genome in such animals. It is furthermore envisaged, that the non-human mammals of the invention can be employed as disease models and for testing therapeutic agents/compositions. Furthermore, the non-human vertebrate or mammal of the invention can also be used for livestock breeding.

In a preferred embodiment, the method of producing a non-human vertebrate or mammal further comprises culturing the cell to form a pre-implantation embryo or introducing the cell into a blastocyst prior to its transfer into the pseudo pregnant female host. Methods for culturing the cell to form a pre-implantation embryo or introducing the cell into a blastocyst are well known in the art and are, for example, described in Nagy *et al.,* loc. cit.

The term "introducing the cell into a blastocyst" as used herein encompasses injection of the cell into a blastocyst as well as fusion of a cell with a blastocyst. Methods of introducing a cell into a blastocyst are described in the art, for example in Nagy *et al*., loc. cit..

The present invention further relates to a non-human vertebrate or mammalian animal obtainable by the above described method of the invention.

In a preferred embodiment, the non-human mammal is selected from the group consisting of rodents, dogs, felides, primates, rabbits, pigs, or cows or the vertebrate is selected from the group consisting of fish such as for example zebrafish, salmon, trout, common carp or coi carp or from avians such as for example chickens, turkeys, pheasants, ducks, geese, quails and ratites including ostriches, emus and cassowaries.

Also described herein is a fusion protein comprising a Tal effector protein and a non-specific cleavage domain of a restriction nuclease. All the definitions and preferred embodiments defined above with regard to the fusion protein in the context of the method of the invention apply *mutatis mutandis.* Furthermore, the present invention also relates to a kit comprising the fusion protein of the invention. The various components of the kit may be packaged in one or more containers such as one or more vials. The vials may, in addition to the components, comprise preservatives or buffers for storage. In addition, the kit may contain instructions for use.

The figures show:

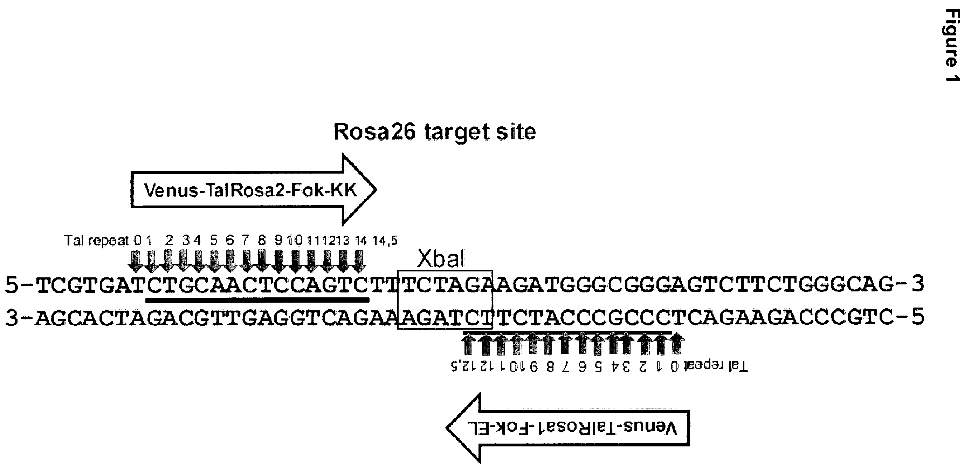

**Figure 1****.** Design of a fusion protein pair in accordance with the present invention, recognizing the mouse genomic Rosa26 locus. Target sequence from the first intron of the mouse Rosa26 locus containing a central Xbal site. The fusion protein Venus-TalRosa2-Fok-KK contains 14 Tal effector motifs (repeat 1-14) fused to the FokI-KK catalytic domain, recognising the underlined target sequence in the upper DNA strand. Fusion protein Venus-TalRosa1-Fok-EL contains 12 Tal effector motifs (repeat 1-12) that recognize the underlined sequence in the lower DNA strand. Both repeat domains are flanked by the invariable first repeat "0" opposing T and the invariable final repeat "12,5" or "14,5". The two fusion proteins are separated by a spacer sequence of 6 basepairs.

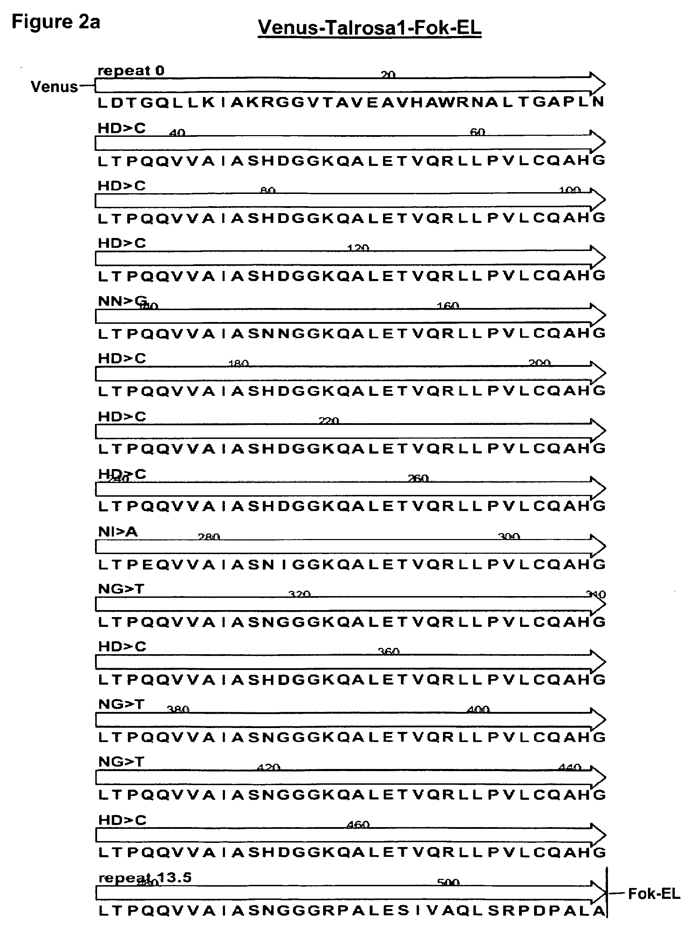

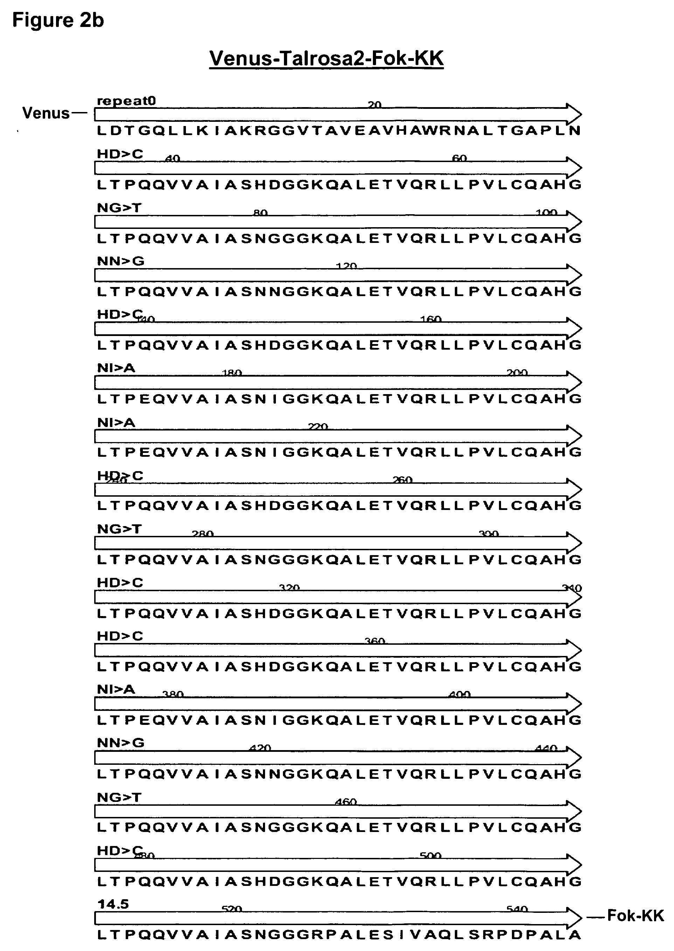

**Figure 2****.** Structure and amino acid sequence of the fusion proteins of the invention recognizing the mouse genomic Rosa26 locus. Shown is the central part of the pair of Rosa26 specific Tal effector DNA-binding domain - nuclease fusion proteins. Each motif comprises 34 amino acids that vary at positions 12 and 13 and determines specificity towards the Rosa26 target sequence, following the code: H12+D13 recognizing C, N12+N13 > G, N12+I13 > A and N12+G13 >T. Both Tal effector DNA-binding domains are N-terminally fused to Venus and C-terminally fused to the Fokl catalytic variant domain Fok-KK or Fok-EL.

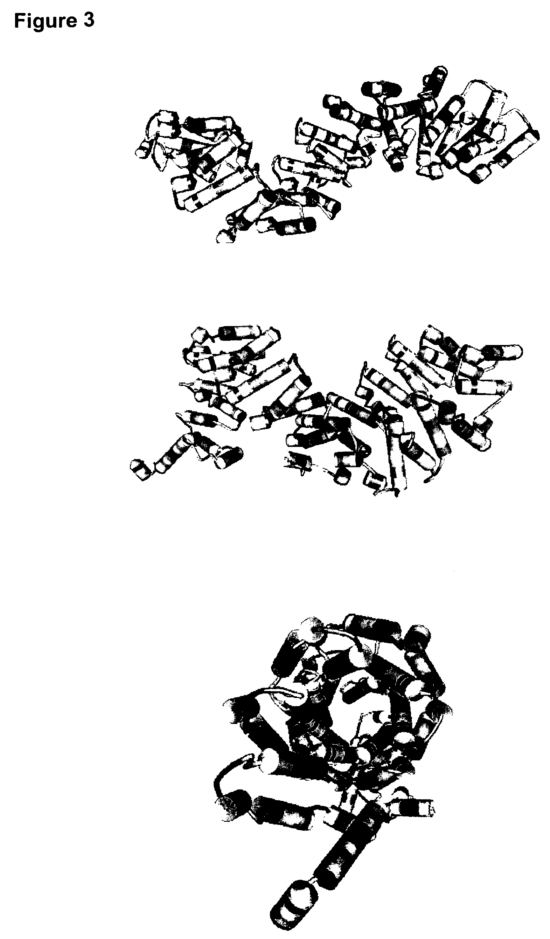

**Figure 3****.** Structural model of a Tal effector DNA-binding domain - nuclease fusion protein of the invention. Structural modeling of an array of 14 Tal effector motifs recognizing a target sequence (GGT-GGC-CCG-GTA-GT) within the mouse Rab38 gene, using the I-Tasser software. As seen in the top (upper graph) and bottom views (middle graph) the Tal effector motifs array in a superhelical structure that could surround a central DNA molecule (not shown). Accordingly, the side view (bottom graph) reveals a free central space to accommodate a substrate DNA molecule. Protein regions forming alpha-helices are shown as schematic tubes; each 34 residue Tal effector motif folds into two helices that are connected by the exposed amino acids at position 12 and 13 that determine DNA sequence specific binding.

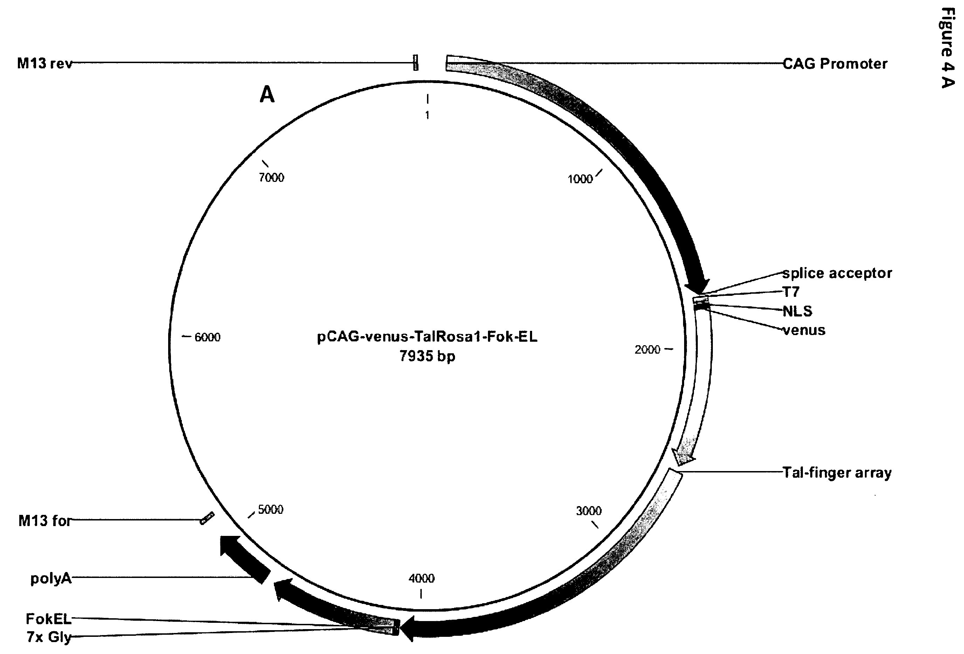

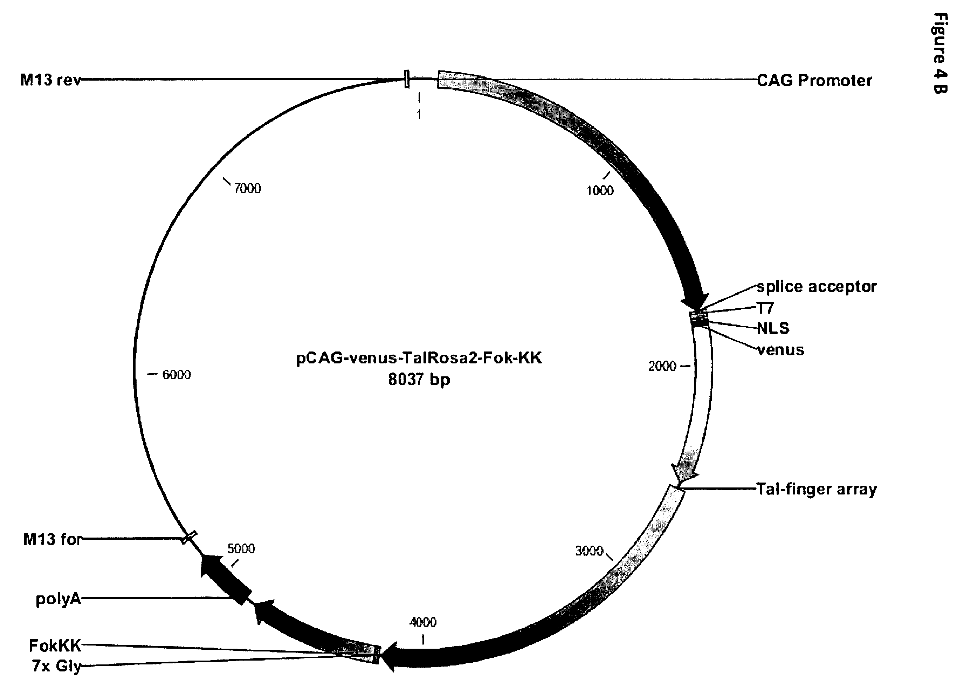

**Figure 4****.** Expression vectors for Tal effector DNA-binding domain - nuclease fusion proteins of the invention. The Rosa26 target sequence specific Tal effector DNA-binding domains TalRosa1 and Talrosa2 are ligated in frame into a plasmid backbone that provides a N-terminal fusion with Venus (including a nuclear localisation signal - NLS) and a C-terminal fusion with the KK or EL mutant of Fokl nuclease, to derive the plasmid pCAG-venus-TalRosa1-Fok-EL (SEQ ID No:2) and pCAG-venus-TalRosa2-Fok-KK (SEQ ID No:4). The Tal effector DNA-binding domain is connected to the Fok domain by a peptide linker of seven glycine residues (7xGly). The coding region of the venus-TalRosa-Fok proteins can be transcribed in vertebrate cells into mRNA from the CAG hybrid promoter and terminated by a polyadenylation signal sequence (polyA) derived from the bovine growth hormone gene. Alternatively mRNA can be transcribed *in vitro* from the phage derived T7 promoter located upstream of the ATG start codon and translated *in vitro* into the venus-TalRosa1-Fok-EL (SEQ ID No:3) and venus-TalRosa2-Fok-KK (SEQ ID No:5) proteins.

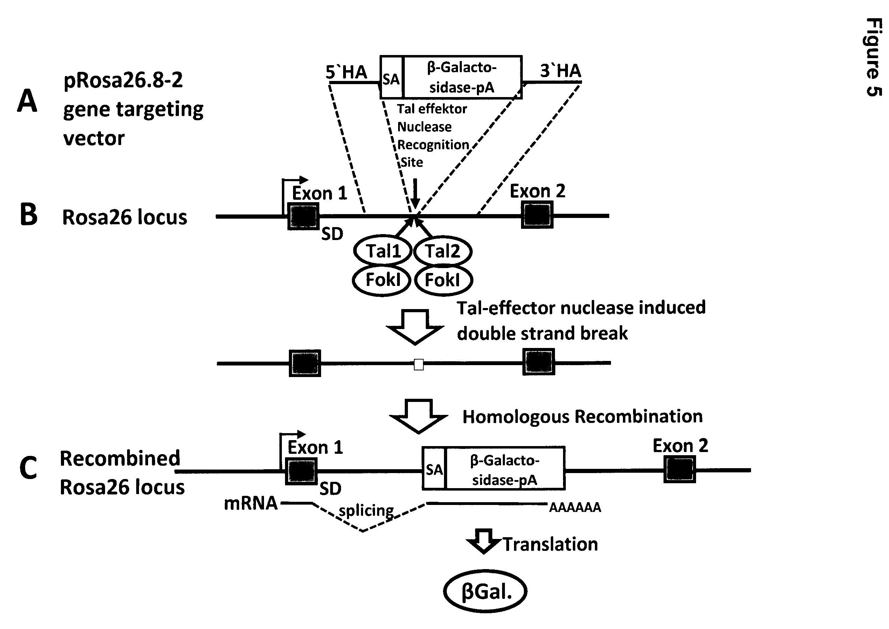

**Figure 5****.** Gene targeting vector pRosa26.8-2 and Tal effector DNA-binding domain - nuclease-assisted homologous recombination at the mouse Rosa26 locus. A: Structure of the gene targeting vector pRosa26.8-2. The 5' and 3' homology regions (5'HR, 3'HR) to the Rosa26 locus are flanking a reporter gene cassette comprising a splice acceptor (SA) sequence, the β-galactosidase coding region and a polyadenylation sequence (pA); B: Genomic structure of the mouse Rosa26 locus. Shown are the first 2 exons of Rosa26 and the Rosa26 promoter (arrow) upstream of exon 1. The homology regions to the pRosa26.8-2 vector within intron 1 are indicated by stippled lines and the target site for the pair of Tal effector DNA-binding domain - nuclease fusion proteins (Fig.1, Fig. 2) is shown by an arrow. Upon a fusion protein-induced double strand break at the target site, homologous recombination with pRosa26.8 is stimulated resulting in a recombined Rosa26 locus; C: Recombined Rosa26 locus. Upon recombination mediated transfer of the reporter gene cassette into the target site for the fusion protein the reporters splice acceptor is spliced to the Rosa26 exon 1 sequence, leading to the production of a mRNA coding for β-galactosidase (βGal.).

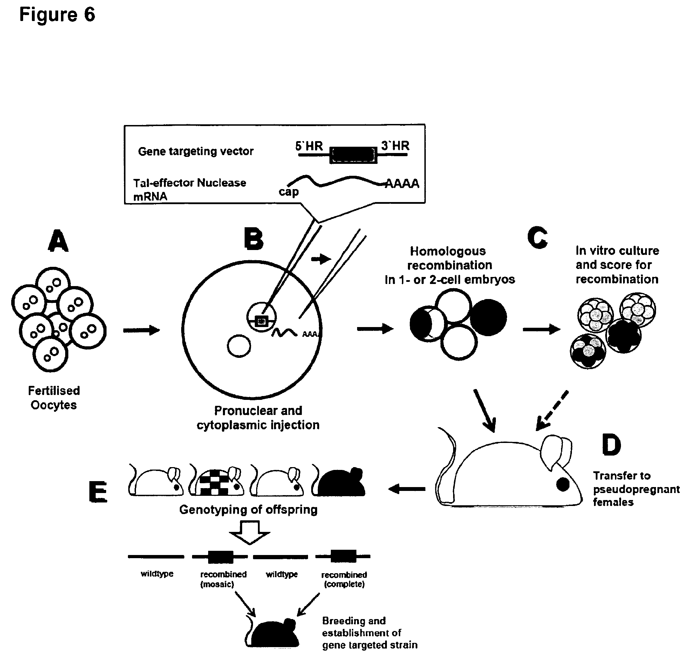

**Figure 6****.** Scheme for the generation of genetically modified mice at the Rosa26 locus by injection of the pRosa26.8-2 gene targeting vector together with mRNA coding for Rosa26 specific fusion protein. A: Fertilised oocytes, collected from superovulated females; B: Microinjection of a gene targeting vector and mRNA coding for Tal effector DNA-binding domain - nuclease fusion proteins into one pronucleus and the cytoplasm of a fertilised oocyte; C: *In vitro* culture of injected embryos and assessment of reporter gene activity. Injected embryos can either directly transferred to pseudopregnant females or after detection of the reporter activity if a live stain is used; D: Pseudopregnant females deliver live offspring from microinjected oocytes, E: The offspring is genotyped for the presence of the induced genetic modification. Positive animals are selected for further breeding to establish a gene targeted strain.
   The examples illustrate the invention.

### Example 1: Construction of Rosa26 specific Tal effector DNA-binding domain - nuclease fusion proteins

### Fusion protein design

To demonstrate the functionality of Tal effector DNA-binding domain - nuclease fusion proteins in mammalian cells we designed a pair of fusion proteins that recognizes a DNA target sequence within the mouse Rosa26 locus (Fig. 1) (SEQ ID NO: 1). The two Tal effector DNA-binding domain - nuclease fusion proteins are intended to bind together to the bipartite target DNA region and to induce a double strand break in the spacer region of the target region to stimulate homologus recombination at the target locus in mammalian cells. The Rosa26 target nucleotides were selected such that the binding regions of the fusion proteins are separated by a spacer of 6 basepairs and each target sequence is preceeded by a T. Following the sequence downstream of the initial T in the 5'>3' direction, base specific Tal effector DNA-binding domain - nuclease fusion proteins were combined together in a N to C terminal order into an array of 12 (TalRosa1) or 14 Tal-fingers (TalRosa2), preceeded by a invariable first (0) and last Tal-finger (12,5;14,5) (Fig. 1). Each Tal effector motif consists of 34 amino acids the position 12 and 13 of which determines the specificity towards recognition of A,G, C or T within the target sequence (Boch, J., et al. 2009 Science 326: 1509-12). To derive Rosa26 specific Tal effector DNA-binding domain - nuclease fusion proteins (Fig.2) we selected the Tal effector motif (repeat) #11 derived from the *Xanthomonas* Hax3 protein (GenBank accession No. AY993938.1 (LTPEQWAIAS**NI**GGKQALETVQRLLPVLCQAHG) with amino acids N12 and 113 to recognize A, the Tal effector motif (repeat) #5 (LTPQQWAIAS**HD**GGKQALETVQRLLPVLCQAHG) derived from the Hax3 protein with amino acids H12 and D13 to recognize C, and the Tal effector motif (repeat) #4 (LTPQQWAIAS**NG**GGKQALETVQRLLPVLCQAHG) from the *Xanthomonas* Hax4 protein (Genbank accession No.: AY993939.1) with amino acids N12 and G13 to recognize T. To recognize a target G nucleotide we used the Tal effector motif (repeat) #4 from the Hax4 protein with replacement of the amino acids 12 into N and 13 into N (LTPQQVVAIAS**NN**GGKQALETVQRLLPVLCQAHG). The base specific DNA-binding domains are preceeded by the invariable first Tal-repeat (LDTGQLLKIAKRGGVTAVEAVHAWRNALTGAPLN) and followed by the last Tal-repeat (LTPEQVVAIASNGGGRPALESIVAQLSRPDPALA) from the Hax3 protein. The DNA-binding domains of the Tal effector proteins recognizing the Rosa26 target sequence were designed in silico using the Vector NTI (Invitrogen) or DNA workbench (CLC) software and combined in frame N-terminally with the GFP variant Venus and C-terminally, via a linker peptide of 7 glycine resiues, with the catalytic domain of Fokl endonuclease to derive the pair of Tal effector DNA-binding domain - nuclease fusion proteins, i.e. venus-TalRosa1-Fok-EL (SEQ ID NO:3) and venus-TalRosa2-Fok-KK (SEQ ID NO:5) (Fig. 2). The catalytic domain of Fokl endonuclease normally acts as a homodimer. To avoid the homodimer formation of a single TalRosa nuclease at nonintended genomic target sequences and thereby to increase the specificity of the Tal effector DNA-binding domain - nuclease fusion protein pair, we used the Fokl mutant domains "KK" and "EL" that preferentially act only as heterodimer (Miller et al. 2007 Nat Biotechnol 25(7): 778-85). In order to model the binding of the fusion proteins of the invention to a DNA target sequence we calculated the 3D structure of a 14 Tal effector motif protein designed to recognize the sequence 5'-GGTGGCCCGGTAGT-3' within the mouse Rab38 gene using the I-Tasser software (Roy et al. 2010 Nat Protoc 5(4): 725-38) and visualized the structure using the Discovery studio software (Accelerys) (Fig.3). According to this structural model the Tal effector motifs fold into a superhelical structure prepared to accomodate a central DNA molecule. Each 34 residue Tal effector motif folds into two helices that are connected by the exposed amino acids at position 12 and 13 that determine DNA sequence specific binding (Fig. 3).

### Expression vectors

To derive vectors for the expression of Tal effector DNA-binding domain - nuclease fusion proteins in mammalian cells the Rosa26 specific coding regions for the Tal effector DNA-binding domain were synthesized by a commercial service provider (Geneart, Regensburg, Germany). The coding DNA fragments for the Tal effector DNA-binding domains TalRosa1 and Talrosa2 were ligated in frame into a plasmid backbone that provides elements for mRNA and protein expression in mammalian cells, specifically a N-terminal fusion with the Venus fluorescent protein (including a nuclear localisation signal - NLS) and a C-terminal fusion with the KK or EL mutant of Fokl nuclease, to derive the plasmids pCAG-venus-TalRosa1-Fok-EL (SEQ ID NO: 2) and pCAG-venus-TalRosa2-Fok-KK (SEQ ID NO: 4). The Tal effector DNA-binding domain is connected to the Fok domain by a peptide linker of seven glycine residues (7xGly). The coding region of the venus-TalRosa-Fok proteins can be transcribed in mammalian cells into mRNA from the CAG hybrid promoter and terminated by a polyadenylation signal sequence (polyA) derived from the bovine growth hormone gene. Alternatively mRNA can be transcribed *in vitro* from the phage derived T7 promoter located upstream of the ATG start codon and translated *in vitro* into the venus-TalRosa1-Fok-EL (SEQ ID NO: 3) and venus-TalRosa2-Fok-KK (SEQ ID NO: 5) proteins.

### DNA cleavage activity of Tal effector DNA-binding domain - nuclease fusion proteins

The designed Tal effector DNA-binding domain - nuclease fusion proteins are tested for function by an *in vitro* nuclease cleavage assay. For this purpose mRNA and protein of the venus-TalRosa-Fok nuclease fusion proteins are produced from the pCAG-venus-TalRosa1-Fok-EL and pCAG-venus-TalRosa2-Fok-KK plasmids using the TnT Quick coupled *in vitro* transcription/translation system from Promega (Madison, WI, USA) following the manufacturers instructions. In an in vitro nuclease assay (Kandavelou 2009 Methods Mol Biol 544: 617-36) a fraction of the synthesized proteins is incubated together with the plasmid pbs-Rosa-targetseq (SEQ ID NO: 7) that contains the Rosa26 target sequence, to assess the cleavage activity of the Tal effector DNA-binding domain - nuclease fusion protein pair. The reaction is analysed for cleavage of the DNA substrate by agarose gel electrophoresis and reveals that the Tal-finger nuclease pair can induce a double strand break within the Rosa26 target sequence.

### Example 2: Tal effector DNA-binding domain - nuclease fusion protein -assisted homologous recombination in fertilized mouse oocytes

With this experiment it is tested whether homologous recombination at the site of a double strand break induced by a Tal effector DNA-binding domain - nuclease fusion protein occurs in fertilised mouse oocytes at a reasonable frequency (>1%). For this purpose we constructed the gene targeting vector pRosa26.8-2 (SEQ ID NO: 6) that inserts a reporter gene cassette into the mouse Rosa26 locus via homology regions. This vector comprises a splice acceptor element, the coding region of β-galactosidase and a polyadenylation sequence, combined with a 1 kb 5'-and 4 kb 3'- homology region derived from the first intron of the *Rosa26* locus (Fig. 5A, B). The *Rosa26* locus is a region on chromosome 6 that has been found to be ubiquitously expressed in all tissues and developmental stages of the mouse and is suitable for transgene expression (Zambrowicz BP, Imamoto A, Fiering S, Herzenberg LA, Kerr WG, Soriano P.; Proc Natl Acad Sci U S A 1997; 94:3789-3794; Seibler J, Zevnik B, Kuter-Luks B, Andreas S, Kern H, Hennek T, Rode A, Heimann C, Faust N, Kauselmann G, Schoor M, Jaenisch R, Rajewsky K, Kuhn R, Schwenk F.; Nucleic Acids Res 2003; 31:e12.). Upon recombination the vector splice acceptor is spliced to the donor site of the Rosa26 transcript such that the fusion transcript codes for β-galactosidase (Fig. 5C).

### A) Results

The linearised targeting vector is microinjected into fertilised mouse oocytes (Fig. 6 A, B) together with *in vitro* transcribed mRNA coding for the pair of Tal effector DNA-binding domain - nuclease fusion proteins (Fig. 2) that recognise the target sequence of Rosa26 (Fig. 1) and induce a double strand break at the insertion site of the reporter gene cassette (Fig. 5B). Upon microinjection, the Tal effector DNA-binding domain - nuclease fusion protein mRNAs are translated into proteins that induce a double strand break at one or both Rosa26 alleles in one or more cells of the developing embryo. This event stimulates the recombination of the pRosa26.8-2 vector with a Rosa26 allele via the homology regions present in the vector and leads to the site-specific insertion of the non-homologous reporter gene cassette into the genome (Fig. 5

C). Depending on the timing of these events recombination may occur within the one cell embryo or later in only a single cell of a 2-cell, 4-cell or 8-cell embryo. To detect such successful recombination events the microinjected zygotes are further cultivated *in vitro* and finally incubated with X-Gal as a β-galactosidase substrate that is converted into a insoluble blue coloured product. In microinjection experiments we observe a high frequency of X-Gal stained embryos indicating the occurrence of homologous recombination at the one cell stage or at a later developmental stage. Since these embryos are fixed before the staining procedure it is not possible to further derive mice from them.

### B) Generation of live mice carrying the reporter gene cassette

In further experiments, the microinjected zygotes are transferred into pseudopregnant females to allow their further development into live mice (Nagy A, Gertsenstein M, Vintersten K, Behringer R., 2003. Manipulating the Mouse Embryo. Cold Spring Harbour, New York: Cold Spring Harbour Laboratory Press). These experiments show that the microinjected zygotes are able to develop into mouse embryos (Fig. 6) and that the integrated reporter gene is expressed. In one such experiment, microinjected zygotes are transferred into a pseudopregnant female mouse and embryos recovered at day 18 of development. The embryos are euthanized, cut into half and one half is stained with X-Gal staining solution as described above. This analysis reveals that one of six embryos is strongly positive for β-Galactosidase reporter gene activity, as indicated by the blue reaction product.

### C) Analysing for successful genomic modification

Without wishing to be bound by the following example, it is envisaged in further experiments to extract genomic DNA from embryonic and newborn, juvenile or adult mice. This DNA can then be analysed for the expected homologous recombination event at the Rosa26 locus by Southern blot analysis using a labelled probe located upstream of the 5' Rosa26 homology arm of the pRosa26 can then be recognised by a band of 11.5 kb while recombined mice can be identified by the presence of an additional band of 3.65 kb.

### Example 3: Material and methods

### Plasmid constructions

The gene targeting vector pRosa26.8-2 (SEQ ID NO: 6) was derived from the vector pRosa26.8 by the removal of a 1.6 kb fragment that contains a pgk-diphtheria toxin A gene. For this purpose pRosa26.8 was digested with EcoRI and Kpnl, the vector ends were blunted by treatment with Klenow and T4 DNA polymerase, and the 12.4 kb vector fragment was re-ligated. pRosa26.8 was derived from pRosa26.1 (Soriano P.; Nat Genet 1999; 21:70-71) by insertion of a I-SceI recognition site into the SacII site located upstream of the 5' Rosa26 homology arm and the insertion of a splice acceptor element linked to the coding region for β-galactosidase and a polyadenylation signal downstream of the 5' homology arm. The expression vectors for Tal-finger nucleases recognising a target site within the first intron of the murine Rosa26 locus (SEQ ID NO: 1) are described in example 1 above.

### Preparation of DNA and RNA for microinjection

Plasmid pRosa26.8-2 is linearised by digestion with I-SceI, precipitated and resolved in injection buffer (10 mM Tris, 0.1 mM EDTA, pH 7.2). Tal effector DNA-binding domain nuclease RNA for injection is prepared from the linearised expression plasmids and transcribed from the T7 promoter using the mMessage mMachine kit (Ambion) according to the manufacturers instructions. The mRNA is further modified by the addition of a poly-A tail using the Poly(A) tailing kit and purified with MegaClear columns from Ambion. Finally the mRNA is precipitated and resolved in injection buffer. Aliquots for injection experiments are adjusted to a concentration of 5 ng /µl of pRosa26.8-2 and 2.5 ng/µl of each Tal effector DNA-binding domain - nuclease fusion protein mRNA.

### Isolation and injection of fertilised oocytes

To isolate fertilised oocytes, males of the C57BL/6 strain are mated to super-ovulated females of the FVB strain. For super-ovulation three-week old FVB females are treated with 2.5 IU pregnant mares serum (PMS) 2 days before mating and with 2.5 IU Human chorionic gonadotropin (hCG) at the day of mating. Fertilised oocytes are isolated from the oviducts of plug positive females and microinjected in M2 medium (Sigma-Aldrich Inc Cat. No. M7167) with the pRosa26.8-2/Venus-TalRosa1/2-Fok-KK/EL mRNA preparation into one pronucleus and the cytoplasm following standard procedures (Nagy A, Gertsenstein M, Vintersten K, Behringer R., 2003. Manipulating the Mouse Embryo. Cold Spring Harbour, New York: Cold Spring Harbour Laboratory Press).

### In vitro culture and X-Gal staining of embryos

For the detection of β-galactosidase activity the microinjected oocytes are further cultivated for 3 days in KSOM medium (Millipore, Cat. No. MR-020-PD) at 37°C/5% CO₂/5% O₂ and fixed for 10 minutes in 4% formaldehyde in phosphate buffered saline (PBS). After washing with PBS the embryos were transferred to X-Gal staining solution (5 mM K3(Fe^{III}(CN)₆) , 5 mM K4(Fe^{II}(CN)₆), 2 mM MgCl₂, 1 mg/ml X-Gal (5-bromo-chloro-3-indoyl-ß-D-galactopyranosid) in PBS) and incubated at 37°C for up to 24 hours.

### References

Bloch, K. D. (2001). "Mapping by multiple endonuclease digestions." Curr Protoc Mol Biol Chapter 3: Unit3 2.
Boch, J., H. Scholze, et al. (2009). "Breaking the code of DNA binding specificity of TAL-type III effectors." Science 326(5959): 1509-12.
Bonas, U., R. E. Stall, et al. (1989). "Genetic and structural characterization of the avirulence gene avrBs3 from Xanthomonas campestris pv. vesicatoria." Mol Gen Genet 218(1): 127-36.
Bradley, A., M. Evans, et al. (1984). "Formation of germ-line chimaeras from embryo-derived teratocarcinoma cell lines." Nature 309(5965): 255-6.
Brinster, R. L., R. E. Braun, et al. (1989). "Targeted correction of a major histocompatibility class II E alpha gene by DNA microinjected into mouse eggs." Proc Natl Acad Sci U S A 86(18): 7087-91.
Capecchi, M. R. (1989). "The new mouse genetics: altering the genome by gene targeting." Trends Genet 5(3): 70-6.
Capecchi, M. R. (2005). "Gene targeting in mice: functional analysis of the mammalian genome for the twenty-first century." Nat Rev Genet 6(6): 507-12.
Cheah, S. S. and R. R. Behringer (2000). "Gene-targeting strategies." Methods Mol Biol 136: 455-63.
Collins, F. S., J. Rossant, et al. (2007). "A mouse for all reasons." Cell 128(1): 9-13.
DeChiara, T. M. (2001). "Gene targeting in ES cells." Methods Mol Biol 158: 19-45.
Doyon, Y., J. M. McCammon, et al. (2008). "Heritable targeted gene disruption in zebrafish using designed zinc-finger nucleases." Nat Biotechnol 26(6): 702-8.
Durai, S., M. Mani, et al. (2005). "Zinc finger nucleases: custom-designed molecular scissors for genome engineering of plant and mammalian cells." Nucleic Acids Res 33(18): 5978-90.
Evans, M. J. and M. H. Kaufman (1981). "Establishment in culture of pluripotential cells from mouse embryos." Nature 292(5819): 154-6.
Geurts, A. M., G. J. Cost, et al. (2009). "Knockout rats via embryo microinjection of zinc-finger nucleases." Science 325(5939): 433.
Gong, M. and Y. S. Rong (2003). "Targeting multi-cellular organisms." Curr Opin Genet Dev 13(2): 215-20.
Gu, H., J. D. Marth, et al. (1994). "Deletion of a DNA polymerase beta gene segment in T cells using cell type-specific gene targeting." Science 265(5168): 103-6.
Hasty, P., A. Abuin, et al. (2000). Gene targeting, principles, and practice in mammalian cells. Gene Targeting: a practical approach. A. L. Joyner. Oxford, Oxford University Press: 1-35.
Hockemeyer, D., F. Soldner, et al. (2009). "Efficient targeting of expressed and silent genes in human ESCs and iPSCs using zinc-finger nucleases." Nat Biotechnol 27(9): 851-7.
Ivarie, R. (2006). "Competitive bioreactor hens on the horizon." Trends Biotechnol 24(3): 99-101.
Kamihira, M., K. Nishijima, et al. (2004). "Transgenic birds for the production of recombinant proteins." Adv Biochem Eng Biotechnol 91: 171-89.
Kandavelou, K. and S. Chandrasegaran (2009). "Custom-designed molecular scissors for site-specific manipulation of the plant and mammalian genomes." Methods Mol Biol 544: 617-36.
Kay, S., J. Boch, et al. (2005). "Characterization of AvrBs3-like effectors from a Brassicaceae pathogen reveals virulence and avirulence activities and a protein with a novel repeat architecture." Mol Plant Microbe Interact 18(8): 838-48.
Kay, S. and U. Bonas (2009). "How Xanthomonas type III effectors manipulate the host plant." Curr Opin Microbiol 12(1): 37-43.
Lai, L. and R. S. Prather (2003). "Creating genetically modified pigs by using nuclear transfer." Reprod Biol Endocrinol 1: 82.
Maeder, M. L., S. Thibodeau-Beganny, et al. (2008). "Rapid "open-source" engineering of customized zinc-finger nucleases for highly efficient gene modification." Mol Cell 31(2): 294-301.
Maeder, M. L., S. Thibodeau-Beganny, et al. (2009). "Oligomerized pool engineering (OPEN): an 'open-source' protocol for making customized zinc-finger arrays." Nat Protoc 4(10): 1471-501.
Miller, J. C., M. C. Holmes, et al. (2007). "An improved zinc-finger nuclease architecture for highly specific genome editing." Nat Biotechnol 25(7): 778-85.
Nagy, A., M. Gertsenstein, et al. (2003). Manipulating the Mouse Embryo. Cold Spring Harbour, New York, Cold Spring Harbour Laboratory Press.
Nothias, J. Y., S. Majumder, et al. (1995). "Regulation of gene expression at the beginning of mammalian development." J Biol Chem 270(38): 22077-80.
Nothias, J. Y., M. Miranda, et al. (1996). "Uncoupling of transcription and translation during zygotic gene activation in the mouse." EMBO J 15(20): 5715-25.
Palmiter, R. D. and R. L. Brinster (1986). "Germ-line transformation of mice." Annu Rev Genet 20: 465-99.
Paques, F. and J. E. Haber (1999). "Multiple pathways of recombination induced by double-strand breaks in Saccharomyces cerevisiae." Microbiol Mol Biol Rev 63(2): 349-404.
Paques and Duchateau (2007). Meganucleases and DNA double-strand break-induced recombination: perspectives for gene therapy. Curr Gene Ther 7(1): 49-66.
Peippo, J., S. Viitala, et al. (2007). "Birth of correctly genotyped calves after multiplex marker detection from bovine embryo microblade biopsies." Mol Reprod Dev 74(11): 1373-8.
Porteus, M. H. and D. Baltimore (2003). "Chimeric nucleases stimulate gene targeting in human cells." Science 300(5620): 763.
Porteus, M. H. and D. Carroll (2005). "Gene targeting using zinc finger nucleases." Nat Biotechnol 23(8): 967-73.
Rouet, P., F. Smih, et al. (1994). "Expression of a site-specific endonuclease stimulates homologous recombination in mammalian cells." Proc Natl Acad Sci U S A 91(13): 6064-8.
Rouet, P., F. Smih, et al. (1994). "Introduction of double-strand breaks into the genome of mouse cells by expression of a rare-cutting endonuclease." Mol Cell Biol 14(12): 8096-106.
Roy, A., A. Kucukural, et al. (2010) "I-TASSER: a unified platform for automated protein structure and function prediction." Nat Protoc 5(4): 725-38.
Santiago, Y., E. Chan, et al. (2008). "Targeted gene knockout in mammalian cells by using engineered zinc-finger nucleases." Proc Natl Acad Sci USA 105(15): 5809-14.
Schwartzberg, P. L., S. P. Goff, et al. (1989). "Germ-line transmission of a c-abl mutation produced by targeted gene disruption in ES cells." Science 246(4931): 799-803.
Seibler, J., B. Zevnik, et al. (2003). "Rapid generation of inducible mouse mutants." Nucleic Acids Res 31(4): e12.
Soriano, P. (1999). "Generalized lacZ expression with the ROSA26 Cre reporter strain." Nat Genet 21(1): 70-1.
te Riele, H., E. R. Maandag, et al. (1992). "Highly efficient gene targeting in embryonic stem cells through homologous recombination with isogenic DNA constructs." Proc Natl Acad Sci U S A 89(11): 5128-32.
Thomas, K. R. and M. R. Capecchi (1987). "Site-directed mutagenesis by gene targeting in mouse embryo-derived stem cells." Cell 51(3): 503-12.
Torres, R. M. and R. Kühn (1997). Laboratory protocols for conditional gene targeting. Oxford, Oxford University Press.
Urnov, F. D., J. C. Miller, et al. (2005). "Highly efficient endogenous human gene correction using designed zinc-finger nucleases." Nature 435(7042): 646-51.
Zambrowicz, B. P., A. Imamoto, et al. (1997). "Disruption of overlapping transcripts in the ROSA beta geo 26 gene trap strain leads to widespread expression of beta-galactosidase in mouse embryos and hematopoietic cells." Proc Natl Acad Sci U S A 94(8): 3789-94.

## Claims

1. A method of modifying a target sequence in the genome of a eukaryotic cell, wherein the cell is an oocyte and wherein the oocyte is not a human oocyte, and wherein the method is not a method for treatment of the animal body by surgery or therapy practised on the animal body, the method comprising the step:
(a) introducing into the cell a fusion protein comprising a DNA-binding domain of a Tal effector protein and a non-specific cleavage domain of a restriction nuclease or a nucleic acid molecule encoding said fusion protein in expressible form, wherein the fusion protein specifically binds within the target sequence and introduces a double strand break within the target sequence.

2. The method of claim 1, wherein the modification of the target sequence is by homologous recombination with a donor nucleic acid sequence further comprising the step:
(b) introducing a nucleic acid molecule into the cell, wherein the nucleic acid molecule comprises the donor nucleic acid sequence and regions homologous to the target sequence.

3. The method of claim 1 or 2, wherein the cell is selected from the group consisting of a mammalian or vertebrate cell.

4. The method of any one of claims 1 to 3, wherein the fusion protein or the nucleic acid molecule encoding the fusion protein is introduced into the cell by microinjection.

5. The method of any one of claims 2 to 4, wherein the nucleic acid molecule of 2(b) is introduced into the cell by microinjection.

6. The method of any one of claims 1 to 5, wherein the nucleic acid molecule encoding the fusion protein in expressible form is mRNA.

7. The method of any one of claims 2 to 6, wherein the regions homologous to the target sequence are localised at the 5' and 3' end of the donor nucleic acid sequence.

8. The method of any one of claims 2 to 7, wherein the regions homologous to the target sequence comprised in the nucleic acid molecule of 2(b) have a length of at least 400 bp.

9. The method of any one of claims 1 to 8, wherein the modification of the target sequence is selected from the group consisting of substitution, insertion and deletion of a least one nucleotide of the target sequence.

10. The method of any one of claims 1 to 9, wherein the cell is from a mammal selected from the group consisting of rodents, dogs, felides, primates, rabbits, pigs, or cows or wherein the cell is from an avian selected from the group consisting of chickens, turkeys, pheasants, ducks, geese, quails and ratites including ostriches, emus and cassowaries or wherein the cell is from zebrafish.

11. A method of producing a non-human vertebrate or mammal carrying a modified target sequence in its genome, the method comprising modifying a eukaryotic cell by the method of any one of claims 1 to 10; and analysing the offspring delivered by the non-human female host to which the cell produced by this method has been transferred, for the presence of the modification.

12. The method of claim 11, further comprising culturing the cell to form a pre-implantation embryo prior to its transfer into the non-human female host.

13. The method of claim 11 or 12, wherein the non-human mammal is selected from the group consisting of rodents, dogs, felides, primates, rabbits, pigs and cows or wherein the vertebrate is selected from the group consisting of fish and avians.

## Patentansprüche

1. Verfahren zum Verändern einer Zielsequenz im Genom einer eukaryontischen Zelle, wobei die Zelle eine Oozyte ist und wobei die Oozyte eine nicht-menschliche Oozyte ist, und wobei das Verfahren kein Verfahren zur chirurgischen oder therapeutischen Behandlung des tierischen Körpers ist, das am tierischen Körper vorgenommen wird, wobei das Verfahren den Schritt umfasst:
(a) Einbringen eines Fusionsproteins, das eine DNA-Bindedomäne eines Tal-Effektor-Proteins und eine nicht-spezifische Spaltungsdomäne einer Restriktionsnuklease umfasst, oder eines Nukleinsäuremoleküls, das das Fusionsprotein in exprimierbarer Form kodiert, in die Zelle, wobei das Fusionsprotein spezifisch innerhalb der Zielsequenz bindet und innerhalb der Zielsequenz einen Doppelstrangbruch einführt.

2. Verfahren nach Anspruch 1, wobei die Veränderung in der Zielsequenz durch homologe Rekombination mit einer Donor-Nukleinsäuresequenz erfolgt, des Weiteren umfassend den Schritt:
(b) Einbringen eines Nukleinsäuremoleküls in die Zelle, wobei das Nukleinsäuremolekül die Donor-Nukleinsäuresequenz und Bereiche umfasst, die homolog zur Zielsequenz sind.

3. Verfahren nach Anspruch 1 oder 2, wobei die Zelle eine Säuger- oder Wirbeltier*-Zelle ist.

4. Verfahren nach einem der Ansprüche 1 bis 3, wobei das Fusionsprotein oder das Nukleinsäuremolekül, das das Fusionsprotein kodiert, mittels Mikroinjektion in die Zelle eingebracht wird.

5. Verfahren nach einem der Ansprüche 2 bis 4, wobei das Nukleinsäuremolekül nach 2(b) mittels Mikroinjektion in die Zelle eingebracht wird.

6. Verfahren nach einem der Ansprüche 1 bis 5, wobei das Nukleinsäuremolekül, das das Fusionsprotein in exprimierbarer Form kodiert, mRNA ist.

7. Verfahren nach einem der Ansprüche 2 bis 6, wobei die Bereiche, die homolog zur Zielsequenz sind, sich an den 5'- und 3'-Enden der Donor-Nukleinsäuresequenz befinden.

8. Verfahren nach einem der Ansprüche 2 bis 7, wobei die in dem Nukleinsäuremolekül nach 2(b) umfassten Bereiche, die homolog zur Zielsequenz sind, eine Länge von mindestens 400 Bp haben.

9. Verfahren nach einem der Ansprüche 1 bis 8, wobei die Veränderung der Zielsequenz der Austausch, das Einfügen oder das Entfernen von mindestens einem Nukleotid der Zielsequenz ist.

10. Verfahren nach einem der Ansprüche 1 bis 9, wobei die Zelle von einem Säuger ausgewählt aus Nagern, Hunden, Katzen, Primaten, Kaninchen, Schweinen oder Kühen stammt oder wobei die Zelle von einem Vogel ausgewählt aus Hühnern, Truthähnen, Fasanen, Enten, Gänsen, Wachteln und Laufvögeln, einschließlich Straußen, Emus und Kasuaren stammt.

11. Verfahren zum Herstellen eines nicht-menschlichen Wirbeltiers oder Säugers, das/der eine veränderte Zielsequenz in seinem Genom trägt, wobei das Verfahren das Verändern einer eukaryontischen Zelle mit dem Verfahren nach einem der Ansprüche 1 bis 10 umfasst, und das Untersuchen der Nachkommen, die von dem nicht-menschlichen, weiblichen Wirt geboren werden, in den die mittels dieses Verfahrens erhaltene Zelle eingebracht wurde, auf das Vorhandensein der Veränderung.

12. Verfahren nach Anspruch 11, des Weiteren umfassend das Kultivieren der Zelle zur Ausbildung eines Präimplantationsembryos, vor deren Einbringen in den nicht-menschlichen, weiblichen Wirt.

13. Verfahren nach Anspruch 11 oder 12, wobei der nicht-menschliche Säuger ausgewählt ist aus Nagern, Hunden, Katzen, Primaten, Kaninchen, Schweinen und Kühen oder wobei das Wirbeltier ausgewählt ist aus Fischen oder Vögeln.

## Revendications

1. Méthode permettant de modifier une séquence cible dans le génome d'une cellule eucaryote, la cellule étant un ovocyte et l'ovocyte n'étant pas un ovocyte humain, et la méthode n'étant pas une méthode de traitement du corps animal par chirurgie ou thérapie pratiquée sur le corps animal, la méthode comprenant l'étape suivante :
(a) l'introduction dans la cellule d'une protéine de fusion comprenant un domaine de liaison de l'ADN d'une protéine effectrice Tal et un domaine de clivage non spécifique d'une nucléase de restriction ou d'une molécule d'acide nucléique codant pour ladite protéine de fusion sous forme exprimable, où la protéine de fusion se lie spécifiquement au sein de la séquence cible et introduit une coupure double brin au sein de la séquence cible.

2. Méthode selon la revendication 1, dans laquelle la modification de la séquence cible est réalisée par recombinaison homologue avec une séquence d'acide nucléique donneuse comprenant en outre l'étape suivante :
(b) l'introduction d'une molécule d'acide nucléique dans la cellule, où la molécule d'acide nucléique comprend la séquence d'acide nucléique donneuse et des régions homologues à la séquence cible.

3. Méthode selon la revendication 1 ou 2, dans laquelle la cellule est choisie dans le groupe constitué d'une cellule de mammifère ou de vertébré.

4. Méthode selon l'une quelconque des revendications 1 à 3, dans laquelle la protéine de fusion ou la molécule d'acide nucléique codant pour la protéine de fusion est introduite dans la cellule par micro-injection.

5. Méthode selon l'une quelconque des revendications 2 à 4, dans laquelle la molécule d'acide nucléique de 2(b) est introduite dans la cellule par micro-injection.

6. Méthode selon l'une quelconque des revendications 1 à 5, dans laquelle la molécule d'acide nucléique codant pour la protéine de fusion sous forme exprimable est de l'ARNm.

7. Méthode selon l'une quelconque des revendications 2 à 6, dans laquelle les régions homologues à la séquence cible sont localisées à l'extrémité 5' et 3' de la séquence d'acide nucléique donneuse.

8. Méthode selon l'une quelconque des revendications 2 à 7, dans laquelle les régions homologues à la séquence cible comprises dans la molécule d'acide nucléique de 2(b) ont une longueur d'au moins 400 pb.

9. Méthode selon l'une quelconque des revendications 1 à 8, dans laquelle la modification de la séquence cible est choisie dans le groupe constitué de la substitution, l'insertion et la délétion d'au moins un nucléotide de la séquence cible.

10. Méthode selon l'une quelconque des revendications 1 à 9, dans laquelle la cellule provient d'un mammifère choisi dans le groupe constitué de rongeurs, chiens, félins, primates, lapins, porcs, ou vaches ou bien où la cellule provient d'un oiseau choisi dans le groupe constitué de poulets, dindes, faisans, canards, oies, cailles et ratites y compris les autruches, les émeus et les casoars ou bien où la cellule provient du poisson zèbre.

11. Méthode de production d'un vertébré ou d'un mammifère non humain portant une séquence cible modifiée dans son génome, la méthode comprenant la modification d'une cellule eucaryote par la méthode selon l'une quelconque des revendications 1 à 10 ; et l'analyse de la descendance délivrée par l'hôte femelle non humain chez lequel la cellule produite par cette méthode a été transférée, pour la présence de la modification.

12. Méthode selon la revendication 11, comprenant en outre la culture de la cellule pour former un embryon de pré-implantation avant son transfert chez l'hôte femelle non humain.

13. Méthode selon la revendication 11 ou 12, dans laquelle le mammifère non humain est choisi dans le groupe constitué de rongeurs, chiens, félins, primate, lapins, porcs et vaches ou bien où le vertébré est choisi dans le groupe constitué de poissons et d'oiseaux.
